(19) Europäisches Patentamt
European Patent Office
Office européen des brevets

(11) EP 2 019 620 B1

(12) # EUROPEAN PATENT SPECIFICATION

(45) Date of publication and mention of the grant of the patent:
**28.03.2012 Bulletin 2012/13**

(21) Application number: **07776887.7**

(22) Date of filing: **07.05.2007**

(51) Int Cl.:
***H01M 8/16*** *(2006.01)* ***H01M 8/04*** *(2006.01)*
***A61B 5/145*** *(2006.01)*

(86) International application number:
**PCT/US2007/011144**

(87) International publication number:
**WO 2007/130694 (15.11.2007 Gazette 2007/46)**

(54) **IMPLANTABLE VOLTAIC CELL**

IMPLANTIERBARES GALAVANISCHES ELEMENT

CELLULE VOLTAÏQUE IMPLANTABLE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **05.05.2006 US 797680 P**

(43) Date of publication of application:
**04.02.2009 Bulletin 2009/06**

(73) Proprietor: **Epps, Spencer J.G.**
**Rockland DE 19732 (US)**

(72) Inventor: **Epps, Spencer J.G.**
**Rockland DE 19732 (US)**

(74) Representative: **Novagraaf Technologies**
**122 rue Edouard Vaillant**
**92593 Levallois-Perret Cedex (FR)**

(56) References cited:
**EP-A1- 1 541 191**
**US-A- 3 837 339**
**US-A- 3 837 339**
**US-A- 3 861 397**
**US-A- 4 294 891**
**US-B1- 6 294 281**
**US-B1- 6 299 757**
**US-B1- 6 466 810**
**US-B2- 7 003 340**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### CROSS-REFERENCE TO RELATED APPLICATIONS

This Application claims benefit of U.S. Provisional Application No. 60/797,680, filed May 5, 2006, .

### FIELD OF THE INVENTION

The invention related to an implantable battery device which operates on an oxidizable biological material. More specifically, the invention relates to a voltaic cell that generates current through the oxidation of glucose. The device is generally applicable to implantable medical devices.

### BACKGROUND OF THE INVENTION

According to the Centers for Disease Control (CDC), there are over 20 million diabetics in the United States alone, and this number is growing at an estimated rate of 11% per year. According to the CDC, billions of dollars are spent annually on treatment of complications resulting from poor diabetic management. The medical literature has shown that strict management of blood sugar within a specific (normoglycemic) range is critical to preventing short-term to mid-term diabetic complications, (*e.g.*, diabetic ketoacidosis) as well as long-term complications (*e.g.*, blindness from diabetic retinopathy, amputation, cardiovascular disease and kidney failure). Standard good management, however, currently requires patients to subject themselves to several painful finger lances a day. Many diabetic patients describe "feeling like a pin cushion," and find this daily monitoring regimen, at best, unpalatable, and in some cases, intolerable.

In many cases, diabetic patients are embarrassed to have to publicly monitor their blood sugar in order to manage their diabetes and the sight of finger-sticking and blood expression makers others uncomfortable, often fearing exposure to communicable disease. Some cultures associate a stigma with diabetes to the point that patients do not want family, friends or neighbors knowing their status as diabetics, for fear of being stigmatized. This often leads to inadequate monitoring of blood sugar and risk of complications associated with poor diabetic management. This greatly contributes to increased morbidity and mortality in the diabetic population. The fear of stigmatization can be particularly acute for teenagers, often resulting in a sudden deterioration of diabetes care with the onset of adolescence and many teenage diabetic deaths each year.

Diabetics, therefore, have long-awaited a simple, convenient, non-invasive, and discrete means to monitor blood sugar.

Several methods are currently available to measure blood glucose, with the most prevalent being finger lancing. Finger lancing involves stabbing of the finger with a small lancet and expressing a drop of blood from the wound which is then blotted onto a test strip (which is then fed into an external glucometer) or the drop of blood is fed directly into the external glucometer. This regimen is painful, inconvenient, and draws attention to the patient's diabetic status.

Other diabetic management devices include insulin pumps. Insulin pumps are essentially glucometers worn by the patient that have a plastic tube that runs directly through an open hole in the patient's abdomen and allows continuous sampling of the glucose levels in the patient's body fluids. The insulin pump continuously monitors the glucose level and injects appropriate insulin doses as determined by a computer program within the insulin pump. This theoretically allows for smooth insulin dosing and good diabetic management. However, there is a possibility that the device could malfunction and expose the patient to serious complications or death from hypoglycaemia (low blood sugar). There is also a risk of infection. These devices are expensive, may not be covered by a patient's insurance, and must be regularly maintained, serviced and calibrated.

EP 1541191 discloses a mediator immobilized on the surface of an anode and a hydrogen and oxygen permeable coating and that the efficiency of a fuel cell can be improved by preventing permeation of substances other than oxygen and hydrogen to react with the cathode.

There remains a need in the art for an inexpensive, implantable glucose meter that reliably provides real time monitoring of a patient's blood glucose concentration.

### SUMMARY OF THE INVENTION

The invention provides an implantable battery device for monitoring blood glucose level without the need for daily finger-lancing. The solution to the problem provides for the more general invention of a battery powered by biological material within bodily fluids.

Thus, the invention provides an implantable battery device comprising at least one voltaic cell that is powered by an oxidizable biological material. As such the invention provides an implantable battery device for use in the body of a subject comprising at least one voltaic cell, each comprising:

- a biologically inert shell (10) defining an inner compartment;
- a cathodic environment within said shell (10) in contact with said ionic solution;
- an anodic environment attached to said outer surface of said shell (10), in contact with bodily fluid wherein said anodic environment accepts electrons from an oxidation reaction with at least one oxidizable biological material in said bodily fluid;
- a connector (18) connecting said anodic environment and said cathodic environment;
- a component that provides resistance (20) disposed along said connector (18) between said anodic environment and said cathodic environment; and
- at least one salt bridge (16),

characterised in that:

said inner compartment containing an ionic solution that mediates a reduction reaction, said shell (10) having an inner surface and an outer surface, said inner surface being in contact with said ionic solution; and
said at least one salt bridge (16) is disposed within said shell (10) selectively permitting passage of hydrogen or oxygen ions between said inner compartment and said bodily fluid to complete the circuit.

The inner compartment contains at least one cathode (cathodic environment) within the ionic solution and the shell has at least one anode (anodic environment) attached to its outer surface that is in contact with bodily fluid of the subject when implanted. The anode accepts electrons from the oxidation reaction when the biological material is oxidized. The voltaic cell has a connector, such as a wire, that connects the anode(s) to the cathodes(s) and has a component that provides resistance between the anode and cathode. The shell also contains at least one salt bridge disposed within the shell that permits the passage of ions between the inner compartment and the bodily fluid, thereby completing the circuit.

The oxidizable biological material may be any biological material found in the subject that can be oxidixed. For example, but not by way of limitation, the material may be glucose, carbohydrates, cholesterols, fatty acids, amino acids, polypeptides, lipids and polynucleotides, nucleotides, nucleotide derivatives, etc. Preferably, the material is glucose.

In some embodiments of the invention, the voltaic cell is adapted to be a sensor for foreign or infectious particles such as viruses, bacteria, parasites, and prions or drugs such as Coumadin ®, synthetic prostacyclins, etc.

In some embodiments the voltaic cell has a plurality of either anodes, cathodes or both. In some embodiments, the cathode is made of a non-magnetic metal (such as, but not limited to silver, aluminum, lead, magnesium, platinum, gold, tin oxide, titanium dioxide, tungsten, metal alloys, non-metallic alloys, semiconductors, and semi-metals). In some embodiments, the anodes are complexed with ammonia molecules to change the potential of the oxidation reaction. In some embodiments the anodes are complexed with catalysts to change the activation energy of the oxidation reaction.

The shell of the voltaic cell may be formed from a biologically inert, impermeable substance containing a plurality of salt bridges, or the shell may be made of a semi-permeable material that allows the passage of ions between the ionic solution of the inner compartment and the surrounding bodily fluid, thereby essentially functioning as a shell with a multitude of salt bridges.

Although the connector in the voltaic cell can be a wire, any connection that allows the conduction of current is a suitable connector in the invention.

In some embodiments, the anodes of the voltaic cell have an inert barrier covering them that permits only the oxidizable biological material to access the anodes to prevent biofouling of the anodes. In some embodiments, this inert layer is on the anodes alone. In other embodiments, a coating may be applied to the outer surfaces of the shell forming an inert barrier on the anodes, allowing only small ions and the oxidizable material to access the anodes and salt bridges.

The component that provides resistance between the anode and the cathode may be a standard resistor, that provides work, or may be a device such as a microradio transmitter that converts the electrical current into radio waves, for example.

In some embodiments, the anodic environment is compartmentalized in the shell and the cathodic environment is disposed on the outer portion of the shell.

An implantable glucometer that allows continuous monitoring of blood glucose in a subject, is disclosed herein. The glucometer is self-sustaining as the oxidation/reduction reaction is self-perpetuating in the body. The glucometer comprises a voltaic cell of the invention adapted to use glucose as the oxidizable biological material and an extracorporeal detection device. The detection device may be placed in proximity of the implanted glucometer and detect a signal generated by the glucometer. The detected signal is automatically correlated to blood glucose levels by the device and the blood glucose level is provided as an output by the device so one may determine the blood glucose level of the subject. The monitoring may be intermittent or continuous, and may be performed in close proximity to the implanted glucometer or remotely. Further, the output from the device may be displayed by the device itself or further transmitted to one or more other devices (such as, but not limited to computer terminals, alarm devices, and telemetric devices such

as cell phones, handheld wireless devices and the like).

In some embodiments, the detection device is an amperemeter that detects electric current. In some embodiments in which the voltaic cell contains a radio transmitter, the detection device is a radio receiver that detects radio waves produced by the glucometer.

A method of monitoring the level of a biological material in the body of a subject by implanting a voltaic cell into the subject and detecting the signal produced by the voltaic device, correlating the signal to the amount of biological material in the bodily fluid of the subject and providing an output of a value for the amount of biological material in the bodily fluid of the subject, is also disclosed herein.

A method for the monitoring of blood glucose in a subject using the glucometer is also disclosed herein. The blood glucose level may be monitored intermittently or continuously. The blood glucose level may be monitored automatically.

A method for powering an implantable medical device in which one or more voltaic cells are operably connected to an implanted device to provide battery power to the device is also disclosed herein. The devices may be powered by a plurality of voltaic cells arranged in series or in parallel.

## BRIEF DESCRIPTION OF THE DRAWINGS

**Figure 1** shows a basic voltaic cell with a single anode and a single cathode. A simple resistor is disposed between the anode and cathode on the connector.

**Figure 2** shows a basic voltaic cell with a single anode and a single cathode. A radio transmitter is disposed between the anode and cathode on the connector.

**Figure 3** shows an embodiment of the voltaic cell powering a device in which a wire disposed between the anodes and cathodes runs to a device to provide power to the device.

**Figure 4** shows an embodiment of the invention in which the voltaic cell is shaped as a cylinder with a central passage. The voltaic cell is shaped to be adapted to be placed in a vessel. The cell contains a plurality of cathodes and anodes. The enlarged portion shows the connection between anode, cathode and connector.

**Figure 5** shows an arrangement of a lattice mesh formed from a plurality of individual voltaic cells (Panel A); a cone-shaped implant formed from the lattice mesh (Panel B); and a U-shaped tube formed from the lattice mesh (Panel C).

**Figure 6** shows anodes covered in a biologically inert layer that prevents macromolecules from bio-fouling the anodes but permits small analytes of interest to access the anodes.

**Figure 7** shows a metabolic voltaic cell in which the anodic environment comprises enzymes and cofactors including oxidases, reductases, dehydrogenases, synthases , enzymes of the citric acid cycle, and the like, as well as Complex 1, Complex II and Coenzyme Q (ubiquinone). The cathodic environment comprises cytochromes c, c, and a + a3, heme-containing porphyrin rings containing iron atoms and Complex III and Complex IV. Connectors connect the anodic and cathodic environments and the connectors contain resistors. Salt bridges are disposed between the anodic and cathodic environments.

**Figure 8** shows a metabolic voltaic cell in which a metabolic environment is disposed above an anodic environment. The metabolic environment comprises enzymes and cofactors including oxidases, reductases, dehydrogenases, synthases , enzymes of the citric acid cycle, and the like, as well as Complex I, Complex II and Coenzyme Q (ubiquinone). The cathodic environment comprises cytochromes b, c, and a + a3, heme-containing porphyrin rings containing iron atoms and Complex III and Complex IV. Connectors connect the anodic and cathodic environments and the connectors contain resistors. Salt bridges are disposed between the anodic and cathodic environments. The anodic environment is separated from the metabolic environment by gated channels operably linked to a receptor for an analyte which extends into the bodily fluid. When an analyte binds to the receptor, the channel opens and electrons from the metabolic environment flow into the anodic environment.

**Figure 9** shows a metabolic voltaic cell in which a metabolic environment is the anodic environment. The metabolic/anodic environment comprises enzymes and cofactors including oxidases, reductases, dehydrogenases, synthases , enzymes of the citric acid cycle, and the like, as well as Complex I, Complex II and Coenzyme Q (ubiquinone). The cathodic environment comprises cytochromes b, c, and a + a3, heme-containing porphyrin rings containing iron atoms, and Complex III and Complex IV. Connectors connect the anodic and cathodic environments and the connectors contain resistors. Salt bridges are disposed between the anodic and cathodic environments. The anodic environment is contained within an impermeable layer which forms a barrier from the bodily fluid. The impermeable layer contains gated channels operably linked to a receptor for an analyte which extends into the bodily fluid. When an analyte binds to the receptor, the channel opens and oxidizable biological material flows into the metabolic/anodic environment and are oxidized.

## DETAILED DESCRIPTION OF THE INVENTION

Any references, patents, patent applications, and/or scientific literature referred to herein evidence the knowledge of those with ordinary skill in the art . In the event of a conflict between any such reference cited herein and the specific teachings of this specification, the specification shall control.

Various definitions are specifically provided in the specification. In general, words shall have the meaning as understood by those of skill in the art. Specifically defined words shall have the meaning given in the definition provided herein. In the event of a conflict between the plain meaning as understood in the art and that specifically provided herein, the definition specifically taught in this specification shall supersede the art-recognized definition. Headings are merely used for convenience and shall not be construed to limit the disclosure in any manner.

Standard reference works setting forth the general principles of biochemistry and electrochemistry known to those of skill in the art include Champe, P. & Harvey, R. LIPPINCOTT'S ILLUSTRATED REVIEWS, 2ND ED. Lippincott, Williams and Wilkins, 1994 and Brown, et al. CHEMISTRY: THE CENTRAL SCIENCE, 10TH EDITION. Pearson Education, 2006.

As used herein, 'oxidizable biological material' shall mean an organic substance that occurs in the body of a subject in which the substance is subject to having the oxidation state of any of its constituent atoms made more positive.

When preferring to the oxidation of glucose herein, this encompasses the oxidation state being made more positive of any atom within the glucose molecule to form any oxidized product of glucose.

When referring to 'reduction' herein, this encompasses the oxidation state of the oxidant (that which is reduced) being made less positive (more negative).

As used herein, "voltaic cell" refers to a battery of separated anodic and cathodic environments connected by a salt bridge. It is also called a Galvanic cell or electrochemical cell.

As used herein, "biocompatible" refers to a state in which the material is non-toxic and a subject's body does not mount an immune response against the material.

As used herein, "subject" refers to an animal, such as a mammal, preferably human.

As used herein, "anode" refers to the electrode where net oxidation occurs. A "anodic environment" refers to an oxidizing environment which serves as an anode.

As used herein, "cathode" refers to the electrode where net reduction occurs. An "cathodic environment" refers to a reducing environment which serves as a cathode.

As used herein, "salt bridge" refers to an ionically conducting passage between separate environments of an electrochemical cell. The salt bridge may contain a conducting material or gel.

As used herein, "semi-permeable" refers to a state in which a material allows certain molecules to flow through the material, but not others.

As used herein, "impermeable" refers to a state in which a material does not allow the flow of any molecules or large atoms through the material.

As used herein, "small ion" refers to $H^+$, $O_2$ (technically, $O_2$ is not an ion but is encompassed by "small ion" as used herein),and any ion necessary to complete the circuit, which can flow through the salt bridge. Small ions do not include macromolecules (such as fibrin, large molecular complexes, enzymes, or cofactors) or larger ions (such as, for example, silver ions).

As used herein, "resistor" refers to an element of an electric circuit with a fixed value of resistance.

As used herein, "amperemeter" refers to a device that detects electrical current.

As used herein, "radio receiver" refers to a device that detects radiowaves emitted by a radio transmitter.

As used herein, "sugar" refers to monosaccharides, aldoses, ketoses, disaccharides, oligosaccharides, homopolysaccharides, heteropolysaccharides, glycosides, complex carbohydrates, etc.

As used herein, "glucose" refers to the monosaccharide, common hexose sugar. "Blood glucose" and "serum glucose" are used synonymously herein.

As used herein, "polypeptide" refers to a natural compound formed from amino acids and includes proteins and protein fragments.

As used herein, "polynucleotide" refers to polymerized nucleic acid and includes DNA and RNA.

As used herein, "nucleotide" refers to nucleotide, nucleosides, or derivatives of either.

As used herein, "cholesterol" refers to the sterol of the formula 10,13-dimethyl-17-(6-methylheptan-2-yl)-2,3,4,7,8,9,11,12,14,15,16,17-dodecahydro-1H-cyclopenta[a]phenanthren-3-ol ($C_{27}H_{46}O$) found in cell membranes and transported in the blood of animals. Enzymatic oxidation of cholesterol forms oxysterols.

As used herein "glucometer" refers to a device that measures blood glucose level of a subject. The glucometer has an implantable portion that "detects" glucose by generating an electric current from the oxidation of glucose. An external device detects the electric current, and through manipulation of the data to convert the electric signal into a correlated value for blood glucose level provides an output that indicates the blood glucose level of the subject in real time. The glucometer may have additional functions such as alarms to indicate hypoglycemia or hyperglycemia, for

example as more fully described herein.

As used herein, "glycotoxicity" refers to tissue toxicity from long-standing hyperglycemia (high blood glucose) secondary to a constellation of pathological metabolic processes, including the conversion of excess glucose to sorbitol. Over time, this and other cytotoxic metabolites irreversibly damage the retina (causing blindness), the vasculature (causing cardiovascular disease, such as stroke, myocardial infarction and impotence), and the nerves (causing diabetic neuropathy with pain or tingling of extremities and eventual anesthesia of these tissues allowing- in the absence of adequate blood flow- infections and tissue damage to accumulate unchecked, eventually resulting in gangrene and amputation).

As used herein "analyte" refers to a substance in biological fluid that can be analyzed. The analyte may be a substance naturally occurring in the body, or may be an infectious agent (e.g., a virus, parasite or bacterium) or a product of an infectious agent. It may also be a drug.

As used herein, "about" refers to +/- 10% of a given reference value.

An implanted voltaic cell that is powered by the oxidation of a biological material is disclosed herein. As a voltaic cell, the oxidation and reduction reactions occur in separate environments.

In a specific embodiment, the voltaic cell is a glucometer. A glucometer is a blood-sugar monitoring device that transmits a diabetic subject's moment-to-moment serum glucose level to an external receiver. After initial implantation, the glucometer of the invention allows for non-invasive measurement without the need for finger lancing. The technology allows for painless glucose monitoring, can be on demand, intermittently, continuously, or assessed conveniently at regular intervals by the patient and medical staff, and can be adapted to calculate required insulin doses, particularly for patients who have difficulty calculating doses on their own. This will allow for exquisite control of blood sugar, therefore forestalling or preventing the sequelae of cumulative glycotoxicity of long-term diabetes. It will also help prevent short-term complications of hyperglycemia, such as diabetic ketoacidosis, a life-threatening spiral of metabolic pathological acidification of the blood. The onset of ketoacidosis can rapidly follow rises in blood glucose from even brief lapses in diabetes control (i.e., missed or skipped insulin doses).

The method provides for monitoring the concentration of an analyte in a subject, or powering a device using current derived from the oxidation of biological materials.

The following discusses the components of the voltaic cell in more detail.

**The Shell of the Voltaic Cell.** The implant is a voltaic cell in which half of the redox reaction is contained in a biologically non-reactive shell. The biological non-reactivity of the shell prevents the subject from mounting an immunological reaction against the shell (such as initiating an allergic reaction, anaphylaxis, fibrosis or immunological destruction of the voltaic cell), while allowing the separation of the anode environment from the cathode environment.

The shell is composed of a biocompatible material such as a polymer (*e.g.*, Teflon, polyethylene glycol (PEG), polyethylene oxide (PEO), PEG/PEO, and the like), plastic treated by coating or impregnation for biocompatibility. Other organic or inorganic biocompatible non-conductive material may also be used, such as treated silicon or glycosaminoglycans/cartilaginous material (*e.g.*, cartilage/hyaluronan) non-immunogenic or rendered so, treated non-immunogenic proteoglycan material, carbon-based molecular scaffolds (*e.g.*, carbon-based nanotubules, fullerene (*e.g.*, buckminsterfullerene structures), and the like.

The shell may be generally impermeable and have at least one salt bridge within the shell. In some embodiments the impermeable shell comprises a plurality of salt bridges. In other embodiments, the shell is semi-permeable, such that the shell's porosity functions as the salt bridge by allowing ions to traverse the shell. The semi-permeable membrane is produced from materials such as, but not limited to the following (in part depending on the biocompatibility, cost, ionic permeability profile, toxicity profile (ideally, non-toxic), and immunogenicity profile): a semi-permeable polymer, agars, a porous inorganic plate (*e.g.*, glass, silicon, *etc.*), an organic membrane (*e.g.*, carbon-based), or lipid-based membrane (charged or uncharged, to modulate membrane permeability). Depending on the material used for the shell, the semi-permeable membrane could be made porous, focally, or diffusely, to allow for small ion permeability (*e.g.*, $H^+$, $O_2$, and any other ion necessary to complete the circuit, but does not allow the flow of macromolecules (such as fibrin, large molecular complexes, enzymes, or cofactors) or larger ions (such as, for example, silver ions).

The shape of the shell (and the voltaic cell in general) is not limited, however, certain shapes may have special utilities in the body of a subject. Thus, the voltaic cell may be tubular, cylindrical, conical, toroidal, U-shaped, V-shaped, rhomboid, spherical, ovoid, or oblong or tesseract-shaped and may be formed into a cluster of cells to form a mesh, a U-shaped mesh, a conical mesh, a toroidal mesh, a tubular mesh, and the like (see **Fig. 5** for illustrative embodiments).

**The Cathode.** The "cathode" is generally a cathodic environment. There is at least one cathode contained within the shell of the voltaic cell in contact with the ionic solution that performs the reduction half-reaction. The cathode is preferably non-magnetic and formed from a metal such as, but not limited to silver, aluminum, lead, magnesium, platinum, gold, tin oxide, titanium dioxide, tungsten, non-magnetic alloys such as stainless steel, brass, semi-metals, semi-conductors, and the like. The cathode may also be formed from a non metal provided it is able to function in the reduction reaction. In some embodiments a voltaic cell comprises a single cathode. In other embodiments, the voltaic cell comprises a plurality of cathodes or a cathodic environment that itself functions as a cathode.

**The Ionic Solutions.** The ionic solution of the voltaic cell may contain silverdiammine complex $[Ag(NH_3)_2^+]$, silver, copper, or iron salts. The use of silver in the ionic solution is more fully described below.

If the oxygen on the carbonyl group of a sugar is unattached to any other structure, the sugar is a reducing sugar, and can serve as the reducing agent of a redox pair in a reduction oxidation reaction to drive the voltaic device. Carbohydrates containing aldehydes or α-hydroxymethyl ketones can be oxidized by Cu(II) ion and are classified as reducing sugars (of which glucose and fructose are examples). They reduce Cu(II) ion to Cu(I).

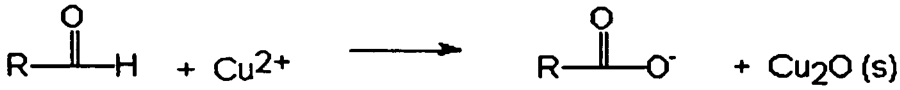

Therefore in the battery, the $Ag^+$ cation of the ionic solution within the shell can be replaced by $Cu^{2+}$, establishing a new redox pair to drive the electrogenerative reaction (this is the basis for Benedict's Test, a means of determining the presence of a reducing sugar in a body fluid or solution, as was once used in the diagnosis of diabetes mellitus). Again, a membrane with enzymatic catalysts at the anode sensor could be applied to facilitate the reaction by lowering the activation energy, but even without an enzymatic substrate, the spontaneous thermodynamics of the reaction prevail. Reducing sugars in the body fluid will be oxidized at the anode in contact with the body fluid, and electrons will flow from the sugar at the anode through a connector to reduce the copper cation at the cathode within the shell. As with other embodiments, a pore (salt bridge, etc.) can be employed to complete the circuit and a device or resister placed in the circuit to, do work, provide power or to send a signal. Modifications or complexes of Cu and catalysts can be used to change the thermodynamics, activation energy, or performance of this votalic cell.

**The Anode.** There is at least one anode at the outer surface of the shell in contact with the bodily fluid in which the oxidation reaction occurs. The anode is preferably formed from a non-magnetic metal such as, but not limited to silver, aluminum, lead, magnesium, platinum, gold, tin oxide, titanium dioxide, tungsten, non-magnetic alloys such as stainless steel, brass, semi-metal, semi-conductors, and the like. The anode may also be formed from a non metal provided it is able to function in the oxidation reaction. In some embodiments a voltaic cell comprises a single anode. In other embodiments, the voltaic cell comprises a plurality of anodes. The anodes may be complexed with an enzyme that catalyzes the oxidation of the biological material of interest. Oxidases are well-known in the art and may be appropriately selected for use in a particular system of choice. The coupling of enzymes to anodes is also known in the art and may be by any means known. For example, the enzyme may be applied in a permeable layer, which may optionally contain stabilizers. It may also be immobilized in this layer.

**The Connector.** The cathode(s) and anode(s) is/are connected to one another by a connector. The connector, may be a wire, for example, but may be any connection that permits the flow of current from the anode to the cathode. The connector is preferably formed from a non-magnetic conductive metal. The connector also comprises a component that provides resistance along the connection. The component may be, for example, a simple resistor. The component may be a radio transmitter, such as a micro-radio that converts electrical current into radiowaves. The component may be another device to be powered by the voltaic cell.

**The Salt Bridge.** The salt bridge is any opening in the shell that permits the passage of small ions. The salt bridge may be a small hole in the shell to selectively allow the passage of the small ions. The shell of the voltaic cell comprises at least one salt bridge. In some embodiments, the shell contains a plurality of salt bridges. In other embodiments the shell is semi-permeable, effectively being a shell containing a multitude of salt bridges.

**Optional Elements.** Optionally, the voltaic cell may further comprise an inert, biologically compatible boundary layer that is disposed over the anode(s) on the outer surface of the shell. The inert boundary layer prevents large molecules from accessing the anodes and only permits the passage of small ions to reach the anode(s). This layer forms a boundary to prevent bio-fouling of the voltaic cell. In some embodiments, the boundary layer only covers the anodes. In other embodiments, the boundary layer is applied as a layer over the surface of the shell. In such a case, the boundary layer is applied such that there is no blocking of the salt bridges of the cell. In general, any boundary layer that prevents bio-fouling may be used. The boundary layer may operate by steric hindrance, size exclusion, surface tension, mass transfer, or any other means known in the art. The boundary layer may be composed of porous polymers that exclude large macromolecules but permit the passage of glucose and other analytes, for example. Materials such as, but not limited to polypropylene, polysulphone, polytetrafluoroethylene (PTFE) and poly(ethylene terephthalate) (PET), or a lipid layer, could be employed.

The voltaic cell may also be coated with an anti-coagulant to prevent clotting of blood around the cell. Anticoagulants are well-known in the art and coating devices and implanted objects with anticoagulants is also known in the art. The anti-coagulant may be applied by bathing, spraying, washing, dipping, immobilizing chemically or otherwise into or incorporating into surface or any other part of device. The anticoagulant may also be an impregnated membrane applied to the device or any part of device. Various anticoagulants are known in the art. Heparin is an example that is

suitable for use in the invention.

The voltaic cell may also be coated with an inhibitor of the deposition of collagen or other matrix proteins to prevent encapsulation of the voltaic cell. Polyethylene glycol (PEG), or phenol structures, are examples of a suitable inhibitor that may be used to inhibit the adherence of proteins from the surface of the voltaic cell.

The voltaic cell may also contain additional electronic or electrophysical components in the circuitry such as, for example, capacitors, semiconductors, transistors, rectifiers, transmitters, receivers, resistors, reference electrodes and the like, depending on the applications of the voltaic cell(s) to be implanted. One of skill in the art is well-versed in circuitry to achieve a desired effect to optimize the performance of the voltaic cell for a given application.

Optionally, a catalyst (enzymatic or otherwise) may be provided at an electrode or at any site of redox reaction to change the activation energy and/or modulate the performance of the voltaic cell. For example, oxidizing enzymes may be complexed on or near the anode of the cell. Examples of enzymes that oxidize biological materials include, but are not limited to:

| Enzyme | Analyte Oxidized |
|---|---|
| glucose oxidase | glucose |
| hexose oxidase | glucose |
| lactate oxidase | lactate |
| 1-amino acid oxidase | 1-methionine |
| 1-amino acid oxidase | 1-phenylalanine |
| D-amino acid oxidase | d-aspartate |
| D-amino acid oxidase | d-glutamate |
| uricase | urate |
| cholesterol oxidase | cholesterol |

Thus, non-limiting examples of enzymes that may be complexed to the anodic portion of the voltaic cell include glucose oxidase, cholesterol oxidase, hexose oxidase, 1-amino acid oxidase, D-amino acid oxidase, uricase, lactate oxidase, choline oxidase, D-amino acid oxidase, alcohol oxidase, uricase, xanthine oxidase, bilirubin oxidase, glutamate oxidase, putrescine oxidase and polyamine oxidase. Further, an optional redox mediator/limiter/amplifier may be provided, as needed or desired.

**External Devices**. The voltaic cell is implanted in the body of a subject, preferably under the skin to allow easy access for external devices to detect signals generated by the voltaic cell. In general, the external devices detect a signal from the voltaic cell and provide an output. In some embodiments, the signal that is detected is current. In other embodiments, the signal that is detected is a radio transmission. In other embodiments, the signal is magnetic flux (which allows the external device to query the implanted device through inductance). In other embodiments the signal is telemetrically received from the implanted voltaic cell. The external devices may include circuitry to calibrate, filter, clean, smooth, amplify or store the data received from the device. The external devices may also analyze the data received and calculate the concentration of the analyte detected by the voltaic cell. For example, the external device may calculate the concentration of blood glucose detected by the voltaic cell based on programmed calculations and/or algorithms performed by the external device. The external device may store the data or further transmit data to other devices (such as a computer, monitor, alarm, cell phone, wireless hand-held devices (e.g., Blackberry™). The functions of the external device are not limited and may be any that are appropriate and useful for the application of the voltaic cell.

In some embodiments, the external device is an amperemeter. In other embodiments the external device is a radio receiver. The external device may monitor the voltaic cell signal on demand, intermittently or continuously. The external device may be placed in close proximity to the voltaic cell (such as by waving a hand-held external device over the area of the subject where the implanted voltaic cell resides). The external device may also be worn by the subject and may take the form of a wristwatch-like device, a collar (particularly for animal subjects), belt or other form conveniently attached to the subject. The external device may also be at a more remote location such as a wall-mounted, tabletop or other station which is close enough to the subject to detect the signal from the voltaic cell. Such devices may be conveniently placed near subjects in a hospital, clinic, nursing home, classroom, home, kennel, and the like. Such devices allow the monitoring of subjects, particularly when the subject cannot reliably monitor themselves (particularly children, the elderly, handicapped, incapacitated, mentally-challenged, and animal subjects).

The external devices may provide a reading of the concentration of the analyte to be measured (*e.g.*, blood glucose) and may include an alarm, instructions for taking a medication based on the reading (*e.g.*, insulin administration),

and the like. As such, the voltaic cell may be coupled with a device that administers drug based on the readings obtained by the device. The drug administration device may be coupled to the voltaic cell or separately connected to the subject and respond to a signal generated from the external device.

The voltaic cells may be used as batteries to power other implanted devices. The voltaic cells may be operably connected to the implanted devices and powered using a ready source of biological material (*e.g.*, glucose). Voltaic cells may be arranged in parallel to increase capacitance and maintain voltage or in series to increase the voltage while maintaining capacitance, depending on the desired application.

**Analytes to be Measured.** The principles of the voltaic cell have broad applicability and may be used as a device to convert the oxidation of a biological material into a detectable signal, and thus, the voltaic cell may be employed as a detection device for a wide variety of analytes. The voltaic cells may detect at least one analyte and may be configured to detect a plurality of analytes. The analytes to be detected include any biological material that can be oxidized at the anode of the voltaic cell. The biological materials include, but are not limited to glucose, sugars, fatty acids, cholesterol, lipids, polynucleotides, amino acids (such as phenylalanine in PKU patients); polypeptides (including for example, hormones such as estrogen, progesterone, testosterone, growth hormone, thyroid hormone and the like (which can be monitored, for example, during hormone therapy, pregnancy, or to detect ovulation); cancer-specific protein markers of cancer cells which can be monitored to detect metastasis or relapse; early markers of disease such as prostate-specific antigen (PSA); and the like) The analytes may also be a drug, such as Coumadin ® or synthetic prostacyclins, etc.

Further, through adapting the voltaic cell to provide a gated channel that opens to expose the anode(s) to an analyte to generate current only when binding of a specific analyte to a receptor operably attached to the channel, the voltaic cell is suitable for detection of foreign or infectious particles, including, for example, viruses, bacteria, parasites and prions. The voltaic cell may be employed, for example to monitor viral load in HIV and other viruses, monitor bacterial/ parasitic infection and antibiotic treatment, and the like.

In one embodiment, for example, a transmembrane spanning protein comprises a channel operably linked to a receptor for viral protein of interest. The membrane with embedded protein coats the anode and prevents the accession of a detectable analyte (such as glucose). When the circulating viral protein reversibly binds to the receptor, a conformational change occurs in the channel and allows glucose to access the anode where it is then oxidized. Current is generated in the voltaic cell, and the external device, which is precalibrated to convert the signal (*e.g.*, current or radiowaves if a radio transmitter is used) to an indication of viral protein concentration, provides a useful output indicating viral load in the subject.

The voltaic cell may also be adapted as a sensor for drugs in the bloodstream of a subject. The sensor may detect at least one drug or metabolite thereof an transmit the data to the external device. This embodiment is useful in monitoring a subject for safe levels of therapeutic drugs such as Coumadin ®, Epoprostenol, pain management drugs and anti-cancer therapies, as well as to monitor the abuse of drugs. In these embodiments, the voltaic cells containing receptors are used in which the receptor detects the presence of the drug or metabolite thereof.

**General Principles of the Voltaic Cell.** A voltaic cell generates current through a redox (reduction-oxidation) reaction, the voltage generation and resultant electric current of which are described by the Nemst equation:

$$E = E^0 - RT/nF \ ln \ Q_c$$

where E is the potential (i.e., the voltage) generated from the reaction. $E^0$ is the standard potential between the two electrodes, *R* is the universal gas constant, *T* is the absolute temperature, *n* is the charge number of the electrode reaction (number of moles of electrons in the reaction), and *F* is the Faraday constant. $Q_c$ represents the ratio of the concentration of the chemical species appearing on the reduced side of the electrode reaction to the concentration of the chemical species appearing on the oxidized side of the electrode reaction (the *In* notation implies the potential generated is related to the natural logarithm of this ratio).

The redox reaction providing the mechanism for electric current generation from serum glucose in the proposed voltaic cell is based on the following oxidation potential of glucose and reduction potential of $Ag^+$:

$$2\,Ag(NH_3)_2^+ + e^- \rightarrow Ag + 2\,(NH_3) \qquad E^0_{red} = 0.373\ V$$

$$+\ C_6H_{12}O_6 + H_2O \rightarrow C_6H_{12}O_7 + 2\,H^+ + 2e^- \qquad E^0_{ox} = 0.600\ V$$

$$2\,Ag(NH_2)_2^+ + C_6H_{12}O_6 + H_2O \rightarrow 2\,Ag + C_6H_{12}O_7 + 2\,NH_4^+ \qquad E^0 = 0.973\ V$$

This reduction-oxidation pairing is known as the Tollens reaction, and is an ancient means of creating a mirrored surface from ionic silver in solution.

The half potentials of voltaic reactions can change when either of the species of the redox pair is modified. For example, the half cell potential of the simple reduction of silver cations ($Ag^+$) is the following:

$$\mathbf{Ag^+ + e^- \rightarrow Ag\ E^0_{red} = 0.800\ V}$$

and the half cell potential of the simple reduction of the glucose molecule is the following:

$$\mathbf{C_6H_{12}O_6 + H_2H_2O \rightarrow C_6H_{12}O_7 + 2H^+ + 2^{e-}\ E^0_{ax} = -0.050\ V}$$

Even though the simple oxidation of the glucose molecule appears to be thermodynamically unfavorable (non-spontaneous because of the negative sign), the overall cell potential of the full reaction, 0.750 V, is thermodynamically favorable and spontaneous, given the overall positivity (positive sign) of the full reaction.

When the silver ion is complexed with $NH_3$. for example, as is the case with the glucometer the half cell potential of the reduction of the silver diammonia cationic complex $[Ag(NH_3)_2{}^+$ differs dramatically:

$$\mathbf{Ag(NH_3)_2{}^+ + e^- \rightarrow Ag + 2\ NH_3\ E^o_{red} = 0.373\ V}$$

The reaction of the modified species, though still spontaneous and thermodynamically favorable, has a significantly smaller magnitude than that of its pure silver cation cousin.

If the reaction were occurring all in one flask, the presence of $NH_3$ molecules would increase the pH of the overall reaction solution, thereby also affecting the half cell potential of the oxidation of glucose. This follows logically from Le Châtelier's Principle. Examining the oxidation reaction of glucose, one can see that the right side of the equation contains $H^+$ (acidic) ions; thus, allowing the reaction to proceed in a high pH milieu (e.g. increased basicity from the presence of NH3 molecules) means that $H^+$ ions will be consumed, and the reaction will be driven to the right. This should mean increased thermodynamic favorability of the oxidation of glucose in the presence of ammonia, and indeed, this is observed:

$$\mathbf{C_6H_{12}O_6 + H_2O \rightarrow C_6H_{12}O_7 + 2\ H^+ + 2\ e^-\ E^*_{ax} = 0.600\ V}$$

As can be seen, the oxidation reaction of glucose is now positive (spontaneous, thermodynamically favorable) in a basic (high pH) milieu. The overall potential of the full redox reaction of the silver complex and glucose in the presence of ammonia is the following:

$$\mathbf{2\ Ag(NH_3)_2{}^+ + C_6H_{12}O_6 + H_2O \rightarrow 2\ Ag + C_6H_{12}O_7 + 2\ NH_4{}^+\ E^o = 0.973\ V}$$

Not only is this still thermodynamically favorable, the cell potential of this reaction is significantly increased compared to that of the redox reaction of the simple silver cation and glucose redox pair.

These are the general principles of the redox reactions which govern the electric potential of the cell. One of skill in the art is well-versed in the ionic solutions and anode/cathode compositions suitable to produce voltaic cells . The standard state cell potentials of half reactions are well known as are ionic solutions and anode/cathodes that would be suitable for use in the invention. The standard-state cell potentials for some common half-reactions are listed in **Table 1.**

**TABLE 1**

| **half-Reaction** | **$E^\circ_{red}$** |
|---|---|
| $K^+ + e^- \rightleftharpoons K$ | -2.924 |
| $Ba^{2+} + 2^{e-} \rightleftharpoons Ba$ | -2.90 |
| $Ca^{2+} + 2^{e-} \rightleftharpoons Ca$ | -2.76 |
| $Na^+ + e^- \rightleftharpoons Na$ | -2.7109 |
| $Mg^{2+} + 2^{e-} \rightleftharpoons Mg$ | -2.375 |
| $H_2 + 2\ e^- \rightleftharpoons 2\ H^-$ | -2.23 |
| $Al^{3+} + 3\ e^- \rightleftharpoons Al$ | -1.706 |
| $Mn^{2+} + 2\ e^- \rightleftharpoons Mn$ | -1.04 |

(continued)

| half-Reaction | $E°_{red}$ |
|---|---|
| $Zn^{2+} + 2 e^- \rightleftharpoons Zn$ | -0.7628 |
| $Cr^{3+} + 3 e^- \rightleftharpoons Cr$ | -0.74 |
| $S + 2 e^- \rightleftharpoons S^{2-}$ | -0.508 |
| $2 CO_2 + 2 H^+ + 2 e^- \rightleftharpoons H_2C_2O_4$ | 1-0.49 |
| $Cr^{3+} + e^- \rightleftharpoons Cr^{2+}$ | -0.41 |
| $Fe^{2+} + 2 e^- \rightleftharpoons Fe$ | -0.409 |
| $Co^{2+} + 2 e^- \rightleftharpoons Co$ | -0.28 |
| $Ni^{2+} + 2 e^- \rightleftharpoons Ni$ | -0.23 |
| $Sn^{2+} + 2 e^- \rightleftharpoons Sn$ | -0.1364 |
| $Pb^{2+} + 2 e^- \rightleftharpoons Pb$ | -0.1263 |
| $Fe^{3+} + 3 e^- \rightleftharpoons Fe$ | -0.036 |
| $2 H^+ + 2 e^- \rightleftharpoons H_2$ | 0.0000... |
| $S_4O_6^{2-} + 2 e^- \rightleftharpoons 2 S_2O_3^{2-}$ | 0.0895 |
| $Sn^{4+} + 2 e^- \rightleftharpoons Sn^{2+}$ | 0.15 |
| $CU^{2+} + e^- \rightleftharpoons Cu^+$ | 0.158 |
| $Cu^{2+} + 2 e^- \rightleftharpoons Cu$ | 0.3402 |
| $O_2 + 2 H_2O + 4 e^- \rightleftharpoons 4 OH^-$ | 0.401 |
| $Cu^+ e^- \rightleftharpoons Cu$ | 0.522 |
| $I_3^- + 2 e^- \rightleftharpoons 3 I^-$ | 0.5338 |
| $MnO_4^- + 2 H_2O + 3 e^- \rightleftharpoons MnO_2 + 4 OH^-$ | 0.588 |
| $O_2 + 2 H^+ + 2 e^- \rightleftharpoons H_2O_2$ | 0.682 |
| $Fe^{3+} + e^- \rightleftharpoons Fe^{2+}$ | 0.770 |
| $Hg_2^{2+} + 2 e^- \rightleftharpoons Hg$ | 0.7961 |
| $Ag^+ + e^- \rightleftharpoons Ag$ | 0.7996 |
| $Hg^{2+} + 2 e^- \rightleftharpoons Hg$ | 0.851 |
| $H_2O_2 + 2 e^- 2 \rightleftharpoons 2 OH^-$ | 0.88 |
| $HNO_3 + 3 H^+ + 3 e^- \rightleftharpoons NO + 2 H_2O$ | 0.96 |
| $Br_2(aq) + 2 e^- \rightleftharpoons 2 Br^-$ | 1.087 |
| $2 IO_3^- + 12 H^+ + 10 e^- \rightleftharpoons I_2 + 6 H_2O$ | 1.19 |
| $CrO_42^- + 8 H^+ + 3 e^- \rightleftharpoons Cr^{3+} + 4 H_2O$ | 1.195 |
| $Pt^{2+} + 2 e^- \rightleftharpoons Pt$ | 1.2 |
| $MnO_2 + 4 H^+ 2 e^- \rightleftharpoons Mn^{2+} + 2 H_2O$ | 1.208 |

(continued)

| half-Reaction | $E°_{red}$ |
|---|---|
| $O_2 + 4\ H^+ + 4\ e^- \rightleftharpoons 2\ H_2O$ | 1.229 |
| $Cr_2O_7^{2-} + 14\ H^+ + 6\ e^- \rightleftharpoons 2\ Cr^{3+} + 7\ H_2O$ | 1.33 |
| $Cl_2(g) + 2\ e^-\ 2 \rightleftharpoons Cl^-$ | 1.3583 |
| $PbO_2 + 4\ H^+ + 2\ e^- \rightleftharpoons Pb^{2+} + 2\ H_2O$ | 1.467 |
| $MnO_4^- + 8\ H^+ + 5\ e^- \rightleftharpoons Mn^{2+} + 4\ H_2O$ | 1.491 |
| $Au^+ + e^- \rightleftharpoons Au$ | 1.68 |
| $H_2O_2 + 2\ H^+ + 2\ e^- \rightleftharpoons 2\ H_2O$ | 1.776 |
| $Co^{3+} + e^- \rightleftharpoons Co^{2+}$ | 1.842 |
| $S_2O_8^{2-} + 2\ e^- \rightleftharpoons 2\ SO_4^{2-}$ | 2.05 |
| $O_3(g) + 2\ H^+ + 2\ e^- \rightleftharpoons O_2(g) + H_2O$ | 2.07 |
| $F_2(g) + 2\ H^+ + 2\ e^- \rightleftharpoons 2\ HF(aq)$ | 3.03 |

With reference to the glucometer version of the voltaic cell, in some embodiments, the reaction is allowed to proceed spontaneously (without ammonia) with an overall cell potential of 0.750 V. However, in practice, this may tend to result in the formation of colloidal silver spheres in solution, rather than the uniform and controlled plating of silver onto the cathode. In addition, it is desirable to have $NH_3$ in cathodic solution as $Ag_2O$ (silver (I) oxide) can be created when elemental silver at the cathode is oxidized by molecular oxygen diffusing into the cathodic environment, over the salt bridge. $Ag_2O$ is minimally soluble in water (0.0013 g/100 ml (20°C) to 0.0017 gm/ 100 ml water) but is more soluble in aqueous solutions of alkali hydroxides, because of the formation of the ion, $Ag(OH)_2^-$. By adding $NH_3$ to the cathodic environment, some portion of the $NH_3$ will react with $H_2O$ to form ammonium hydroxide ($NH_4OH$), maintaining the oxidized silver in solution to reenter the cycle of reduction at the cathode, thus helping perpetuate the reaction. It is unclear whether the formation of colloidal silver would interfere over time with the function of the device, but, without wishing to be bound to any particular theory of operability, it is presently believed that silver metal plating onto the cathode is preferable.

In other embodiments, the reduction of, and plating of silver onto the cathode is employed. In these embodiments, the ionic solution within the shell contains a basic compound such as containing high ammonia. Other basic species may be used, but this will likely change the thermodynamics of the reaction, such that one of skill in the art would select the solutions to suit the particular application, and such skill is well within the ambit of the skilled artisan. The glucose oxidation half reaction occurs in the near neutral pH of the blood. This results in an overall cell potential of about 0.323 V. This is still a spontaneous reaction, although the thermodynamic favorability of this reaction is of lower magnitude.

In other embodiments of the invention, a membrane is deposited or incorporated at the anode that is impregnated with or complexed with $NH_3$ molecules or some other molecular species to mediate the thermodynamics of the redox reaction. In the case of $NH_3$, for example, complexing this species to the anode (or the membrane incorporated with the electrode) would result in a higher overall cell potential: 0.973 V.

In other embodiments, the anode(s) could have $NH_3$ complexed to its (their) surface(s) (or some other basic molecule or ion) to allow the oxidation of glucose to occur in a high pH environment, but allow for the formation of colloidal silver by not providing ammonia inside the shell. This results in a predicted overall cell potential of the reaction of 1.4 V.

Thus, it is possible to modulate the potential of the half reactions by modifying the pH of the milieu at the anode, the cathode, neither, or both.

Glucose is an energy-rich biological material that is well suited for the voltaic cell . However, the invention is not limited to harnessing energy through the oxidation of glucose. The body has many energy-rich materials that can be used by adapting the voltaic cell, and the power of any energy-rich biological molecule can be harnessed to provide electrical energy by separating biochemical redox pairs. If the electrons that are naturally transferred among biological redox pairs are allowed to flow through a connector, a current is generated.

The metabolism of biological materials are facilitated by well-understood enzymatic catalysts and biochemical reactions. These include, for example:

(a) fatty acids (triacylglycerols, prostaglandins, steroids, *etc.*) through β-oxidation, decarboxylation, hydrolysis/hydroxylation, *etc.*

(b) carbohydrates (monosaccharides, aldoses, ketoses, disaccharides, oligosaccharides, homopolysaccharides, heteropolysaccharides, glycosides, complex carbohydrates, *etc.*) through hydrolysis, conversion to acetyl CoA, oxidation, glycolysis, the citric acid cycle, *etc.*

(c) amino acids (glucogenic, ketogenic; oxaloacetate-generating; α-ketoglutarate-generating; pyruvate and fumarate-generating; succinyl Co, acetyl CoA, and acetoacetyl CoA-generating, etc.), and proteins (including glycoproteins, lipoproteins, etc.) through transamination, deamination/oxidative deamination, oxidative decarboxylation, dehydrogenation, *etc.*; and

(d) other energy-rich biological molecules or metabolites such as glycerols (from the mobilization of stored fat, etc.), pyruvate/lactate, ATP/ADP, ketone bodies (acetoacetate, β-hydroxybutarate, acetone), through oxidation, hydrolysis, conversion, etc.

The metabolism of these molecules donates electrons to nicotinic adenine dinucleotide ($NAD^+$) and flavin adenine dinucleotide (FAD) through (reductases, dehydrogenases, synthases, *etc.*) to form the reduced, electron (and energy) rich molecules of NADH and $FADH_2$, respectively.

These reduction oxidation reactions are the first step of the body's primary means of creating ATP (a major energy currency molecule used to perform work in the cells). Once NADH and $FADH_2$ have been created from metabolism of fatty acids, amino acids and carbohydrates (and other substrates, comprising but not restricted to those above), they donate their electrons down the electron transport chain through coenzymes to oxygen, an avid electron acceptor. This forms the basis of oxidative phosphorylation, or cellular respiration, by which the oxidation of fatty acids, amino acids and carbohydrates to $CO_2$ and $H_2O$ transfers electrons to $O_2$, which is reduced to $H_2O$.

This forms the basis of a biological redox pair for use in the voltaic device. The anode would comprise immobilized, well described, reversible enzymes for the metabolism of fatty acids, amino acids and carbohydrates (reductases, dehydrogenases, synthases, enzymes of the citric acid cycle, etc.), and Complex I, Complex II and coenzyme Q of the inter-membrane space of the mitochondria, in contact with the body fluid:

**Complex I:** Comprises $NAD^+$/NADH, FMN/$FMNH_2$, NADH dehydrogenase

**Complex II:** Comprises $NAD^+$/NADH, FAD/$FADH_2$, fumarate/succinate, succinate dehydrogenase, acyl CoA dehydrogenase

**Coenzyme Q:** A quinine derivative (also known as ubiquinone, because of its ubiquity in living systems) oxidizes (takes electrons from) both $FMNH_2$ and $FADH_2$ and transfers them to Complexes IIII and IV.

The hydrogen ions (cationic and anionic) are readily available in the blood stream and small enough to perfuse the reaction arena to allow for the transfer of electrons through the trading of hydrogen ions between reactants. The FMN/$FMNH_2$ pair is a reversible intermediate in the electron transport chain that oxidizes NADH to $NAD^+$ (by reducing FMN to $FMNH_2$). The electrons from this redox reaction are then transferred from $FMNH_2$ to Coenzyme Q, through the oxidation of $FMNH_2$ to FMN and the reduction of CoQ to $CoQH_2$. Likewise, $FADH_2$ is oxidized to FADH to reduce CoQ to $CoQH_2$.

Complexes III and IV, and cytochrome c of the electron transport chain would make up the cathode. The components of this system of complexes are the cytochromes b, c, and a + $a_3$, containing heme groups made of porphyrin rings containing an iron atom that is reversibly converted from its ferric ($Fe^{3+}$) to its ferrous state ($Fe^{2+}$) through redox reaction, thus serving as a reversible carrier of electrons.

**Complex III:** Comprises Cytochrome b, which receives electrons from (is reduced by) Coenzyme Q, oxidizing $CoQH_2$ to CoQ.

**Cytochrome c:** Transfers electrons from Complex III to Complex IV

**Complex IV:** Comprises cytochrome a + $a_3$ and accepts electrons from (is reduced by) cytochrome c, oxidizing the metals groups of cytochrome c. Cytochrome a + $a_3$ is the only cytochrome in which the heme iron has a free ligand that can readily react with $O_2$. This cytochrome also contains bound copper atoms, required for the reaction. Electrons are transferred to $O_2$, reducing it to $H_2O$, which oxidizes the metal groups of cytochrome a + $a_3$.

As in other embodiments, the $O_2$ molecule (and hydrogen ions) will naturally perfuse through the pores/salt bridges of the shell and be available for reduction to $H_2O$ at the cathode. The $O_2$ molecule will not be appreciably reduced at the anode outside the shell, though oxygen is richly available there, because (1) there is no cytochromic iron moiety free

ligand to react with it, and (2) the electron transport chain is downhill; once the electrons have been passed via hydrogen ions to subsequent intermediaries of the chain, they cannot travel backward up the chain as this is thermodynamically unfavorable. Electrons will have flowed down to cytochrome a + $a_3$ providing a ready supply of raw electrons to react with oxygen at the cathode. By separating the redox pair and facilitating the irreversible flow of electrons into the initial components of the electron transport system, oxygen at the cathode becomes an electron well, and is readily available there to receive them.

As in other embodiments, the anodic environment in contact with the blood stream is separated from the cathodic environment and joined by a connector. The flow of electrons through the connector generates a current. The cathodic environment should be impermeable to components of Complexes I and II, and ideally metabolic intermediaries, though $O_2$ and small ions such as protons and $Cl^-$, should be able to pass freely.

The Nernst thermodynamics of this reaction are favorable, which means it is spontaneous and can be harnessed to generate electric power:

| Redox Reaction | $E_9$ (Standard Reduction Potential) |
|---|---|
| $NADH \rightarrow NAD^+$ | 0.32 |
| $FADH_2 \rightarrow FAD$ | 0.22 |
| ½ $O_2 \rightarrow H_2O$ | 0.82 |
| **Total Reaction** | **1.36** |

The voltaic cell may be constructed compartmentalizing the anodic environment, comprising electron donors such as NADH, $FADH_2$, $FMNH_2$, antioxidants such as glutathione or ascorbate (ascorbic acid, or vitamin C), or active reductants such as Sodium borohydride ($NBH_4$). The cathodic environment would now be in contact with the blood stream, whereby the voltaic is powered by reduction of oxidants in the blood stream (or body fluid), with electrons passing from the electron donorslantioxidants/reductants through the connector to the oxidants, thus generating a current. In this embodiment, it would be ideal to sequester the anodic environment from more electronegative species such as $O_2$ or oxygen radicals, $H_2O_2$, nitrogen oxide species ($-NO_2$/-NO), $Fe^{3+}$, $Ca^{3+}$, $Mg^{3+}$, etc., in order to allow the orderly flow of electrons through the connector.

As is well known in the art, a voltaic cell can also be run backward to re-oxidize oxidants previously used in the cathodic environment. This is referred to as electrolysis, whereby an outside power source, for example, in this case magnetic inductance, is used to pump electrons "uphill." When this reverse reaction begins, what is referred to as the "cathodic environment" and the "anodic environment" are now reversed, by convention. For clarity, we remember electrons always move from the anode toward the cathode, in both cases.

Certain embodiments of the invention will now be described with reference to the drawings.

A voltaic cell, in basic format, is shown in **Fig. 1.** The voltaic cell has a biocompatible shell **10** defining a compartment which encloses a cathode **12** in an ionic solution. The shell **10** has at least one salt bridge **16** that allows the passage of ions between the ionic solution and the bodily fluid outside the shell **10.** The voltaic cell also has at least one anode **14** positioned on the outer surface of the voltaic cell. The anode **14** is connected to the cathode **12** by a connector **18** (such as a wire). A component that provides resistance **20** is positioned between the anode and the cathode. The large arrows represent bodily fluid surrounding the voltaic cell.

In practice, the exterior surface of the implanted cell will be in contact with bodily fluid (*e.g.*, blood) of the subject. The blood glucose is oxidized at anode **14** and electrons flow down the connector **18** to the cathode **12.** $Ag^+$ ions in the ionic solution within the shell are reduced and plated onto the cathode **12.** Salt bridges **16** are provided that allow small ions to flow through the shell to complete the circuit. In this environment, oxygen diffuses across the salt bridge **16** and reoxidizes the silver at cathode **12,** and the silver returns to the ionic solution as $Ag^+/[Ag(NH_3)_2{}^+]$. As the reactions occur, electrons flow from the anode **14** to the cathode **12** along the connector **18.** A device providing resistance (such as a resistor) **20** is disposed along the connector **18.** As current flows through the voltaic cell, an external device positioned outside of the body of the subject can detect the current.

In another embodiment shown in **Fig. 2,** a voltaic cell, in basic format, is shown. The voltaic cell has a biocompatible shell **10** defining a compartment which encloses a cathode **12** in an ionic solution. The shell **10** has at least one salt bridge **16** that allows the passage of small ions between the ionic solution and the bodily fluid outside the shell **10.** The voltaic cell also has at least one anode **14** positioned on the outer surface of the voltaic cell. The anode **14** is connected to the cathode **12** by a connector **18** (such as a wire). In this case, the component that provides resistance **21** is a micro radio transmitter positioned between the anode and the cathode. The large arrow represents passage of bodily fluid surrounding the voltaic cell. In practice, the current flowing through the voltaic cell is converted to radiowaves which are emitted from the device. These radiowaves can be detected using a radio receiver which is positioned outside

of the body of the subject.

**Fig. 3** shows another embodiment of the voltaic cell used as a battery containing an impermeable shell **10,** a plurality of anodes **14,** a plurality of cathodes **12,** salt bridges **16,** and connectors **18** connecting the anodes **14** and cathodes **12.** The anodes and cathodes are connected by connectors **18** that have a device disposed along the connector that provides resistance **20** and is powered by the voltaic cell.

**Fig. 4** shows an embodiment of the voltaic cell adapted for use in a vessel. The shell **10** has at least one salt bridge **16** that allows the passage of small ions between the ionic solution and the bodily fluid outside the shell **10.** The shell **10** is shaped as a tube comprising a cylinder with a central passage for blood flow. The interior compartment of the shell contains a plurality of cathodes **12.** The passage contains a plurality of anodes **14** positioned on the surface of the shell **10** positioned on the aspect in contact with the flow of blood. The inset shows that cathodes **12** are connected to anodes **14** by a connector **18** having an element providing resistance **20** on the connector **18.** The voltaic cell has salt bridges **16** disposed on the shell **10** to allow flow of ions between the blood stream and the inner compartment containing the ionic solution.

**Fig. 5** shows an embodiment in which a plurality of voltaic cells is interwoven to create a lattice mesh **(Panel A).** Each voltaic cell of the mesh comprises a plurality of anodes (represented by black circles) which are connected to internal cathodes by connectors. The mesh may be implanted directly into the body of the subject or formed into a particular shape, adapted for implantation at a desired site within the body. **Fig. 5 (Panel B)** shows a cone-shaped device formed from the mesh. **Fig. 5 (Panel C)** shows a U-shaped tube device formed from the mesh. **Fig. 5 (Panel D)** shows a cup-shaped tube device formed from the mesh.

**Fig. 6** shows view of anodes **14** on the surface of a shell **10** wherein the anodes **14** are covered with a biologically inert boundary material **30** that forms a barrier to large bio-fouling molecules, but permits small molecules and analytes to enter pores **32** and contact the anodes **14.** The anodes **14** are connected to cathodes **12** with connectors **18** which have resistors **20.**

**Figure 7** represents an embodiment of the invention in which the anodic environment contains metabolic enzymes to form a "metabolic environment." In this case, the environment is a metabolic/anodic environment and comprises the metabolic enzymes (Complex I, Complex II, Coenzyme Q and any oxidoreductases, transferases, hydrolases, lyases, isomerases and ligases useful in metabolizing the oxidizable biological material from the bodily fluid) in contact with the bodily fluid (dotted line indicates access to bodily fluid) such that metabolism at the metabolic/anodic environment occurs constitutively. The cathodic environment comprises Complex III, cytochrome c and Complex IV to mediate the reduction portion. Connectors with resistors nm between the metabolic/anodic environment and the cathodic environment, and salt bridges are provided to allow the flow of small ions to complete the circuit.

**Figure 8** represents an embodiment in which the metabolic environment is separated from the anodic environment by an impermeable layer which contains gated channels operably linked to receptors for an analyte of interest. In this embodiment, free analytes in the bloodstream come into contact with the receptor and bind to the receptor. When no analyte is bound (left receptor), the channel is closed and no electrons of metabolism in the metabolic environment are able to flow into the anodic environment. When the analyte binds the receptor, the channel opens (right channel), allowing electrons to flow into the anodic environment, and down the connectors to the cathodic environment. Salt bridges allow flow of small ions to complete the circuit.

**Figure 9** represents an embodiment of the invention in which the anodic environment contains metabolic enzymes to form a "metabolic environment." In this case, the environment is a metabolic/anodic environment and comprises the metabolic enzymes (Complex I, Complex II, Coenzyme Q and any oxidoreductases, transferases, hydrolases, lyases, isomerases and ligases useful in metabolizing the oxidizable biological material from the bodily fluid) is sequestered from the bodily fluid by an impermeable barrier (solid line indicates no access to bodily fluid) such that metabolism at the metabolic/anodic environment does not occur constitutively. The impermeable barrier contains gated channels operably linked to receptors for an analyte of interest. In this embodiment, free analytes in the bloodstream come into contact with the receptor and bind to the receptor. When no analyte is bound (left receptor), the channel is closed and no oxidizable biological material is able to flow into the metabolic/anodic environment. When the analyte binds the receptor, the channel opens (right channel), allowing oxidizable biological material to flow into the metabolic/ anodic environment, and electrons generated flow down the connectors to the cathodic environment. Salt bridges allow flow of small ions to complete the circuit.

Although certain presently preferred embodiments of the invention have been specifically described herein, it will be apparent to those skilled in the art to which the invention pertains that variations and modifications of the various embodiments shown and described herein may be made without departing from the spirit and scope of the invention. The examples provided are merely illustrative of the invention and should not be construed as limiting.

## EXAMPLES

### Example 1

In an embodiment of the invention, the shell contains a non-magnetic cathode (such as silver) in an aqueous ammonia solution. A non-magnetic wire connects the cathode to an anode outside of the shell (to prevent short-circuiting of the cell and abolition of current generation). The summed electric potential of the reaction (*$E^o$ = 0.323 V*) represents the overall thermodynamics favorability of the reaction of the oxidation of glucose and the reduction of $Ag^+$ from the aqueous diamminesilver complex $[Ag(NH_3)_2^+]$ in solution to elemental silver (pure silver metal).

The oxidization of glucose is a highly thermodynamically favorable reaction because of the high potential chemical energy stored in carbohydrates (it is precisely because of this that glucose is the body's primary means of generating energy for life). It is through the voltaic mechanism described above that the chemical energy is converted to electric energy. Thus, as blood flows around (or through, in the case of tubular or mesh shapes) the device, glucose in the blood passing by the anode is oxidized to gluconic acid (*i.e.*, electrons are stripped from glucose) resulting in the reduction of the silver. Electrons thus flow, from the glucose molecules, across the connector connecting the anode and the cathode, where $Ag^+$ is reduced and plated onto the cathode. The device needs only exposure to blood, or other body fluid (such as urine, interstitial fluid, peritoneal fluid *etc.*), which can be a capillary, for example. It does not need to be implanted in a vein or an artery.

Ions, such as protons ($H^+$ ions) are allowed to flow through a semi-permeable membrane in the shell, which serves as a salt bridge to complete the circuit, or through salt bridges engineered into the cell. In this environment, oxygen will tend to diffuse across the salt pore to reoxidize the elemental silver reduced in the redox reaction which will return to solution as $Ag^+/[Ag(NH_3)_2^+]$, and allow the circuit to run perpetually. This does not represent a perpetual energy device; the energy for this recharging of the system derives from the high energy of the glucose oxidation reaction (from glucose readily available in the bloodstream, particularly in diabetes) and the thermodynamically favorable oxidation of silver by elemental oxygen. The thermodynamically favorable oxidation of silver by elemental oxygen is why silver naturally develops a patina over time.

The signal may be transmitted in a variety of ways. For example, the increase in current generated in the voltaic cell from increased oxidation of glucose due to increased serum glucose concentration is detected by an external amperemeter (current detector), which is calibrated to interpret incremental increase in current as specific incremental increase in serum glucose concentration **(Fig. 1).** In this case, the current generated by the glucose meter device (effectively, a bio-voltaic or living-system integrated battery device, by design), can be measured by an external amperemeter. This would measure magnetic flux based on the change of current strength in the implant, just under the skin. The strength of the magnetic field, and thus the magnitude of the magnetic flux, varies proportionately to the magnitude of the current (current is defined as moving charges; moving charges set up magnetic field; a change in magnetic field can be measured over a distance by a change in magnetic flux, which is the basis of common magnetometers).

Alternatively, a micro-radio is built into the voltaic cell and powered by the circuit generated from the oxidation of glucose **(Fig. 2).** This radio emits higher frequencies radio waves ("higher frequency radio waves" are higher energy waves; this energy increase will come from the higher current (from increasing available electric potential energy derived from increasing glucose concentration, as glucose is a high energy molecule). The higher frequency radio waves are emitted as the current increases secondary to increased serum glucose concentration and thus, increased redox activity. These waves are detected by an external radio receiver, calibrated to translate increases in radio frequency into information on the incremental increases in serum glucose concentration being measured by the implant. This can be achieved using basic, solid-state semiconductor physics. For example, an analogue micro-transistor can be used to stabilize and amplify radio frequency derived from an alternating electric current generated by the device (based on the oxidation of glucose). A radio signal is emitted when a wire carries an alternating current. A simple transmitter results from connecting this system to an antenna. Radio receivers which can be used to receive a radio transmission and be adapted to calculate analyte levels and provide an output are known in the art. An example is found in US 2005/02457299, U.S. Patent No. 6,585,644 .

Harmonic frequency filtration can be achieved through combinations of inductors and capacitors. A bipolar junction transistor is applicable as measurement of current is desired. Otherwise, a field effect transistor, to measure voltage, high gain or digital transistor could be employed, as well. Other types of transistors (dual gates, transistor arrays and combinations, MOSFET, IGBT, IGFET, *etc.*) of various semiconductor materials (compound, alloys, *etc.*) may also be used.

In some embodiments, the transistor is part of an integrated circuit, in others it is desirable to use an individual transistor of the device, where the current generated from the oxidation of blood (serum) glucose at the anode flows from the anode to the input terminal of the transistor and modulates the conductivity between the other two terminals, thereby modulating the flow of current between these terminals, allowing for amplification and translation of current changes into radio frequency. In the example of a general transistor, the greater the current (in the case of the glucometer

implant, progressively higher blood glucose external to the device would generate a progressively higher continuous current) the greater the conductivity between the terminals, and thus the translatability of current into radio frequency, which can be measured by an external radio frequency receiver, a widely available commodity, for display as precise moment to moment glucose level and allow for calculation by this external device of the appropriate insulin dose, once calibrated to the patient.

A digital transistor would allow for the device to act as a microprocessor, which could further process the information on glucose-mediated current generation into various forms of data, within the device, and allow for transmission of these digital data to the external device. Alternatively, a microprocessor may be part of the external device. In an alternative embodiment, microprocessors are included in both the implanted device and the external device.

In cases using detection of voltage, or telemetric detection such as through radio frequency or other wireless communication, an external receiver is calibrated to interpret increases in blood glucose. From this, a receiver (*e.g.*, amperemeter, radio receiver) could be programmed to derive, by common and simple clinical algorithms, the appropriate insulin dose for the attendant serum glucose concentration. Because of slight physiological and metabolic differences between patients, the receiver will be calibrated at time of installation, and its accuracy can be monitored instantaneously by checking against glucometer readings done by finger stick at any time, representing an important safety measure.

The device will be installed by a clinician, similar to installation of depo birth control devices, inconspicuously under the skin of the arm or abdomen. When the patient wishes to check his or her blood sugar, he or she will hold the receiver over the implant, which will read out the implant's electric current or radio frequency at that moment as a glucose level.

**Example 2**

A voltaic cell glucometer is subcutaneously implanted into a diabetic patient who is able to monitor and regulate his or her own insulin levels. The glucometer emits radiowaves which continuously monitor the patient's glucose levels. The signal is received by a radio receiver worn as a wristwatch-type receiver that displays the patient's blood glucose level. The wristwatch receiver also displays the time of day and is equipped with an alarm to alert the patient when his or her glucose level drops to a point at which the patient becomes hypoglycemic, or when an insulin does is required to treat or prevent hyperglycemia.

**Example 3**

A voltaic cell glucometer is subcutaneously implanted into a diabetic patient who is unable to monitor and regulate his or her own insulin levels. The patient is an incapacitated person in the hospital. The glucometer emits radiowaves which continuously monitor the patient's glucose levels. The signal is received by a radio receiver installed near the patient's hospital bed (or alternatively worn in close proximity to the implanted device). The signals emitted by the implanted glucometer are received by the external receiver which continuously calculates and displays the patient's blood glucose level. In addition the receiver further transmits the data by wireless communication to a computer at the nurse's station. The computer at the nurse's station displays the patient's information, vital signs and blood glucose level. The computer may provide an alert if the patient becomes hypoglycemic, or hyperglycemic, or indicate when insulin is required. In addition, the radio receiver on or near the patient may also be equipped with an alarm to indicate hypoglycemia or hyperglycemia. In this way the nurse or medical staff can attend to a diabetic patient in an incapacitated state without the need to draw blood and test glucose levels and thereby maintain better control of a patient's blood glucose level more conveniently and efficiently.

**Example 3**

A voltaic cell having cholesterol oxidase complexed to the surface of the anode of the voltaic cell is implanted into a patient having severe hypercholesterolemia. The voltaic cell is in contact with the patient's blood and cholesterol flowing past the anodes of the cell is oxidized and the electrons flow through the connector to the cathode within the shell of the voltaic cell. The current generated is converted to high frequency radio waves which are received by an external radio receiver which is programmed and calibrated to calculate and display the patient's blood cholesterol level. The patient's cholesterol level is monitored to assess the patient's response to dietary restrictions and therapy with cholesterol-lowering drugs, to help prevent heart attacks and strokes.

**Example 4**

A diabetic dog has a voltaic glucometer implanted under the skin of the scruff of the neck. The voltaic cell glucometer detects the dog's blood glucose level which generates current in the voltaic cell. The current is detected by

an amperemeter which is worn as a dog collar. The amperemeter is equipped with an alarm that alerts the dog's owner when the dog becomes hypoglycemic or hyperglycemic and displays the blood glucose level. The dog's owner is then alerted to feed the dog or call the veterinarian in hypoglycemia, or administer insulin to the dog in hyperglycemia.

The specific embodiments described herein contain descriptions which are generally applicable to other embodiments of the invention and are not limited solely to the specific embodiment described.

## Claims

1. An implantable battery device for use in the body of a subject comprising at least one voltaic cell, each comprising:

    - a biologically inert shell (10) defining an inner compartment;
    - a cathodic environment within said shell (10) in contact with said ionic solution;
    - an anodic environment attached to said outer surface of said shell (10), in contact with bodily fluid wherein said anodic environment accepts electrons from an oxidation reaction with at least one oxidizable biological material in said bodily fluid;
    - a connector (18) connecting said anodic environment and said cathodic environment;
    - a component that provides resistance (20) disposed along said connector (18) between said anodic environment and said cathodic environment; and
    - at least one salt bridge (16),

    **characterised in that**:

    said inner compartment containing an ionic solution that mediates a reduction reaction, said shell (10) having an inner surface and an outer surface, said inner surface being in contact with said ionic solution; and
    said at least one salt bridge (16) is disposed within said shell (10) selectively permitting passage of hydrogen or oxygen ions between said inner compartment and said bodily fluid to complete the circuit.

2. The implantable battery device of claim 1 wherein said cathodic environment comprises at least one cathode (12).

3. The implantable battery device of claim 1 wherein said anodic environment comprises at least one anode (4).

4. The implantable battery device of claim 2 wherein said anodic environment comprises at least one anode (14).

5. The implantable battery device of claim 1 wherein said oxidizable biological material is selected from the group consisting of carbohydrates, cholesterols, fatty acids, amino acids, polypeptides, lipids, hormones and polynucleotides.

6. The implantable battery device of claim 5 wherein said carbohydrate is glucose.

7. The implantable battery device of claim 5 wherein said hormone is selected from the group consisting of estrogen, progesterone, testosterone, growth hormone and thyroid hormone.

8. The implantable battery device of claim 5 wherein said polypeptide is prostate-specific antigen or a cancer-specific polypeptide.

9. The implantable battery device of claim 1 wherein said ionic solution comprises diamminesilver complex $[Ag(NHs)_2J^+$, silver, copper, or iron salts

10. The implantable battery device of claim 1 wherein said voltaic cell comprises a plurality of cathodes (12).

11. The implantable battery device of claim 1 wherein said voltaic cell comprises a plurality of anodes (14).

12. The implantable battery device of claim 11 wherein said voltaic cell comprises a plurality of cathodes (12).

13. The implantable battery device of claim 1 wherein said shell comprises a plurality of salt bridges (16).

14. The implantable battery device of claim 1 wherein said connector (18) is a wire.

**15.** The implantable battery device of claim 1 wherein said cathode (12) is a non-magnetic metal.

**16.** The implantable battery device of claim 2 wherein said cathode (12) is selected from the group consisting of silver, aluminum, lead, magnesium, platinum, gold, tin oxide, titanium dioxide, tungsten, non-magnetic alloys, semiconductors, semimetals and organometallic complexes.

**17.** The implantable battery device of claim 1 wherein said shell (10) is formed from an impermeable material.

**18.** The implantable battery device of claim 1 wherein said shell (10) is formed from a semi-permeable material.

**19.** The implantable battery device of claim 1 further comprising an inert barrier having selective permeability to exclude bio-fouling molecules wherein said barrier covers said anode.

**20.** The implantable battery device of claim 19 wherein said barrier comprises a layer that covers said outer surface of said shell.

**21.** The implantable battery device of claim 1 wherein said component that provides resistance (20) is selected from the group consisting of a microradio or microprocessor.

**22.** The implantable battery device of claim 3 wherein said anode is complexed with ammonia (NH3) molecules.

**23.** The implantable battery device of claim 3 wherein said anode (14) comprises an enzyme that oxidizes said oxidizable biological material.

**24.** The implantable battery device of claim 23 wherein said enzyme is selected from the group consisting of glucose oxidase, cholesterol oxidase, hexose oxidase, 1 -amino acid oxidase, D-amino acid oxidase, uncase, lactate oxidase, choline oxidase, alcohol oxidase, uncase, xanthine oxidase, bilirubin oxidase, glutamate oxidase, and polyamine oxidase.

**25.** The implantable battery device of claim 1 further comprising a metabolic environment disposed on the outer surface of said shell or layered over said anodic environment wherein said metabolic environment comprises a plurality of metabolic enzymes and cofactors that oxidize a plurality of oxidizable biological materials.

**26.** The implantable battery device of claim 25 wherein said metabolic environment comprises metabolic enzymes and cofactors selected from the group consisting of oxidoreductases, transferases, hydrolases, lyases, isomerases, ligases, coenzymes, cofactors, and prosthetic groups.

**27.** The implantable battery device of claim 26 wherein said plurality of metabolic enzymes and cofactors comprise oxidases, reductases, dehydrogenases, synthases, citric acid cycle enzymes, $NAD^+$/NADH, FMN/$FMNH_2$, NADH dehydrogenase, FAD/FADH2, fumarate/succinate, succinate dehydrogenase, acyl CoA dehydrogenase, ubiquinone.

**28.** The implantable battery device of claim 27 wherein said cathodic environment comprises cytochromes b, c, and a + $3_3$, iron-containing porphyrin, and ubiquinone.

**29.** The implantable battery device of claim 25 wherein a single layer comprises said metabolic environment and said anodic environment.

**30.** The implantable battery device of claim 25 wherein said metabolic environment is disposed in a layer on an outer portion of said anodic environment and is in contact with said bodily fluid, wherein said anodic environment is separated from said metabolic environment by an impermeable boundary layer, wherein said boundary layer comprises gated channels that are operably connected to receptors for an analyte, said receptors extending from said channels to said bodily fluid, wherein binding of said analyte to said receptor causes said channels to open and permit flow electrons to anodic environment.

**31.** The implantable battery device of claim 28 wherein said anodic environment comprises a impermeable boundary layer between said anodic environment and said bodily fluid, wherein said boundary layer comprises gated channels that are operably connected to receptors for an analyte, said receptors extending from said channels to said bodily

fluid, wherein binding of said analyte to said receptor causes said channels to open and permit flow of said oxidizable biological material into said anodic environment.

**32.** The implantable battery device according to claim 1, wherein said anodic environment comprises electron donors, antioxidants or active reductants; a cathodic environment.

**33.** The implantable battery device according to claim 1, wherein said electron donors comprise NADH, $FADH_2$, and $FMNH_2$-.

**34.** The implantable battery device according to claim 1, wherein said antioxidants comprise glutathione or ascorbate.

**35.** The implantable battery device according to claim 1, wherein said active reductants comprise sodium borohydride.

**36.** The implantable battery device of claim 1 wherein the oxidizable material is glucose, further comprising a detection device, wherein said detection device detects a signal from said first component and provides a value that correlates with blood glucose level in said subject.

**37.** The implantable battery device of claim 36 wherein said detection device is an amperemeter.

**38.** The implantable battery device of claim 36 further comprising an inert barrier layer having selective permeability for small ions covering said anode.

**39.** The implantable battery device of claim 36 wherein said anodic environment comprises glucose oxidase.

**40.** The implantable battery device of claim 39 wherein said glucose oxidase is complexed with at least one anode.

**41.** The implantable battery device of claim 36 wherein said anodic environment comprises ammonia.

## Patentansprüche

**1.** Implantierbare Batterieeinheit für die Verwendung im Körper eines Subjekts, umfassend wenigstens eine voltaische Zelle, jeweils umfassend:

- eine biologisch inerte Hülle (10), die ein inneres Kompartiment definiert;
- eine kathodische Umgebung innerhalb der Hülle (10) in Kontakt mit der ionischen Lösung;
- eine anodische Umgebung, die an der äußeren Oberfläche der Hülle (10) befestigt ist, in Kontakt mit Körperfluid, wobei die anodische Umgebung Elektronen von einer Oxidationsreaktion mit wenigstens einem oxidierbaren biologischen Material in dem Körperfluid akzeptiert;
- einen Konnektor (18), der die anodische Umgebung und die kathodische Umgebung verbindet;
- eine Komponente, die Widerstand (20) bereitstellt und entlang des Konnektors (18) zwischen der anodischen Umgebung und der kathodischen Umgebung angeordnet ist; und
- wenigstens eine Salzbrücke (16),

**dadurch gekennzeichnet, dass**:

das innere Kompartiment eine ionische Lösung enthält, die eine Reduktionsreaktion vermittelt, wobei die Hülle (10) eine innere Oberfläche und eine äußere Oberfläche aufweist, wobei die innere Oberfläche in Kontakt mit der ionischen Lösung steht; und

die wenigstens eine Salzbrücke (16) innerhalb der Hülle (10) angeordnet ist und selektiv die Passage von Wasserstoff- oder Sauerstoffionen zwischen dem inneren Kompartiment und dem Körperfluid erlaubt, um den Kreis zu schließen.

**2.** Implantierbare Batterieeinheit gemäß Anspruch 1, wobei die kathodische Umgebung wenigstens eine Kathode (12) umfasst.

**3.** Implantierbare Batterieeinheit gemäß Anspruch 1, wobei die anodische Umgebung wenigstens eine Anode (14) umfasst.

4. Implantierbare Batterieeinheit gemäß Anspruch 2, wobei die anodische Umgebung wenigstens eine Anode (14) umfasst.

5. Implantierbare Batterieeinheit gemäß Anspruch 1, wobei das oxidierbare biologische Material ausgewählt ist aus der Gruppe bestehend aus Kohlenhydraten, Cholesterolen, Fettsäuren, Aminosäuren, Polypeptiden, Lipiden, Hormonen und Polynukleotiden.

6. Implantierbare Batterieeinheit gemäß Anspruch 5, wobei das Kohlenhydrat Glucose ist.

7. Implantierbare Batterieeinheit gemäß Anspruch 5, wobei das Hormon ausgewählt ist aus der Gruppe bestehend aus Estrogen, Progesteron, Testosteron, Wachstumshormon und Schilddrüsenhormon.

8. Implantierbare Batterieeinheit gemäß Anspruch 5, wobei das Polypeptid prostataspezifisches Antigen oder ein krebsspezifisches Polypeptid ist.

9. Implantierbare Batterieeinheit gemäß Anspruch 1, wobei die ionische Lösung Diamminsilber-Komplex $[Ag(NHs)_2]^+$-, Silber-, Kupfer- oder Eisensalze umfasst.

10. Implantierbare Batterieeinheit gemäß Anspruch 1, wobei die voltaische Zelle eine Vielzahl von Kathoden (12) umfasst.

11. Implantierbare Batterieeinheit gemäß Anspruch 1, wobei die voltaische Zelle eine Vielzahl von Anoden (14) umfasst.

12. Implantierbare Batterieeinheit gemäß Anspruch 11, wobei die voltaische Zelle eine Vielzahl von Kathoden (12) umfasst.

13. Implantierbare Batterieeinheit gemäß Anspruch 1, wobei die Hülle eine Vielzahl von Salzbrücken (16) umfasst.

14. Implantierbare Batterieeinheit gemäß Anspruch 1, wobei der Konnektor (18) ein Draht ist.

15. Implantierbare Batterieeinheit gemäß Anspruch 1, wobei die Kathode (12) ein nichtmagnetisches Metall ist.

16. Implantierbare Batterieeinheit gemäß Anspruch 2, wobei die Kathode (12) ausgewählt ist aus der Gruppe bestehend aus Silber, Aluminium, Blei, Magnesium, Platin, Gold, Zinnoxid, Titandioxid, Wolfram, nichtmagnetischen Legierungen, Halbleitern, Halbmetallen und organometallischen Komplexen.

17. Implantierbare Batterieeinheit gemäß Anspruch 1, wobei die Hülle (10) aus einem undurchlässigen Material hergestellt ist.

18. Implantierbare Batterieeinheit gemäß Anspruch 1, wobei die Hülle (10) aus einem halbdurchlässigen Material hergestellt ist.

19. Implantierbare Batterieeinheit gemäß Anspruch 1, ferner umfassend eine inerte Barriere mit selektiver Durchlässigkeit zum Ausschließen von biologisch verunreinigenden Molekülen, wobei die Barriere die Anode bedeckt.

20. Implantierbare Batterieeinheit gemäß Anspruch 19, wobei die Barriere eine Schicht umfasst, die die äußere Oberfläche der Hülle bedeckt.

21. Implantierbare Batterieeinheit gemäß Anspruch 1, wobei die Komponente, die Widerstand (20) verleiht, ausgewählt ist aus der Gruppe bestehend aus einem Mikroradio und einem Mikroprozessor.

22. Implantierbare Batterieeinheit gemäß Anspruch 3, wobei die Anode mit Ammoniak($NH_3$)-Molekülen komplexiert ist.

23. Implantierbare Batterieeinheit gemäß Anspruch 3, wobei die Anode (14) ein Enzym umfasst, welches das oxidierbare biologische Material oxidiert.

24. Implantierbare Batterieeinheit gemäß Anspruch 23, wobei das Enzym ausgewählt ist aus der Gruppe bestehend aus Glucoseoxidase, Cholesteroloxidase, Hexoseoxidase, 1-Aminosäureoxidase, D-Aminosäureoxidase, Uncase,

Lactatoxidase, Cholinoxidase, Alkoholoxidase, Uncase, Xanthinoxidase, Bilirubinoxidase, Glutamatoxidase und Polyaminoxidase.

**25.** Implantierbare Batterieeinheit gemäß Anspruch 1, ferner umfassend eine metabolische Umgebung, die an der äußeren Oberfläche der Hülle angeordnet ist oder über die anodische Umgebung geschichtet ist, wobei die metabolische Umgebung eine Vielzahl von metabolischen Enzymen und Cofaktoren umfasst, die eine Vielzahl von oxidierbaren biologischen Materialien oxidieren.

**26.** Implantierbare Batterieeinheit gemäß Anspruch 25, wobei die metabolische Umgebung metabolische Enzyme und Cofaktoren umfasst, ausgewählt aus der Gruppe bestehend aus Oxidoreduktasen, Transferasen, Hydrolasen, Lyasen, Isomerasen, Ligasen, Coenzymen, Cofaktoren und prosthetischen Gruppen.

**27.** Implantierbare Batterieeinheit gemäß Anspruch 26, wobei die Vielzahl von metabolischen Enzymen und Cofaktoren Oxidasen, Reduktasen, Dehydrogenasen, Synthasen, Citronensäurezyklus-Enzyme, $NAD^+$/NADH, FMN/$FMNH_2$, NADH-Dehydrogenase, FAD/FADH2, Fumarat/Succinat, Succinatdehydrogenase, Acyl-CoA-Dehydrogenase, Ubichinon umfasst.

**28.** Implantierbare Batterieeinheit gemäß Anspruch 27, wobei die kathodische Umgebung Cytochrome b, c und a + $3_3$, eisenhaltiges Porphyrin und Ubichinon umfasst.

**29.** Implantierbare Batterieeinheit gemäß Anspruch 25, wobei eine einzelne Schicht die metabolische Umgebung und die anodische Umgebung umfasst.

**30.** Implantierbare Batterieeinheit gemäß Anspruch 25, wobei die metabolische Umgebung in einer Schicht auf einem äußeren Abschnitt der anodischen Umgebung angeordnet ist und in Kontakt mit dem Körperfluid steht, wobei die anodische Umgebung von der metabolischen Umgebung durch eine undurchlässige Grenzschicht getrennt ist, wobei die Grenzschicht gesteuerte Kanäle umfasst, die operativ mit Rezeptoren für einen Analyten verbunden sind, wobei sich die Rezeptoren von den Kanälen zu dem Körperfluid erstrecken, wobei Binden des Analyten an den Rezeptor bewirkt, dass sich die Kanäle öffnen und das Strömen von Elektronen zu der anodischen Umgebung erlauben.

**31.** Implantierbare Batterieeinheit gemäß Anspruch 28, wobei die anodische Umgebung eine undurchlässige Grenzschicht zwischen der anodischen Umgebung und dem Körperfluid umfasst, wobei die Grenzschicht gesteuerte Kanäle umfasst, die operativ mit Rezeptoren für einen Analyten verbunden sind, wobei sich die Rezeptoren von den Kanälen zu dem Körperfluid erstrecken, wobei Binden des Analyten an den Rezeptor bewirkt, dass sich die Kanäle öffnen und das Strömen des oxidierbaren biologischen Materials in die anodische Umgebung erlauben.

**32.** Implantierbare Batterieeinheit gemäß Anspruch 1, wobei die anodische Umgebung Elektronendonatoren, Antioxidationsmittel oder aktive Reduktanten; eine kathodische Umgebung umfasst.

**33.** Implantierbare Batterieeinheit gemäß Anspruch 1, wobei die Elektronendonatoren NADH, $FADH_2$ und $FMNH_2$-umfassen.

**34.** Implantierbare Batterieeinheit gemäß Anspruch 1, wobei die Antioxidationsmittel Glutathion oder Ascorbat umfassen.

**35.** Implantierbare Batterieeinheit gemäß Anspruch 1, wobei die aktiven Reduktanten Natriumborhydrid umfassen.

**36.** Implantierbare Batterieeinheit gemäß Anspruch 1, wobei das oxidierbare Material Glucose ist, ferner umfassend eine Nachweiseinheit, wobei die Nachweiseinheit ein Signal von der ersten Komponente nachweist und einen Wert bereitstellt, der mit dem Blutglucosespiegel in dem Subjekt korreliert.

**37.** Implantierbare Batterieeinheit gemäß Anspruch 36, wobei die Nachweiseinheit ein Amperemeter ist.

**38.** Implantierbare Batterieeinheit gemäß Anspruch 36, ferner umfassend eine inerte Barriereschicht, die selektive Durchlässigkeit für kleine Ionen aufweist und die Anode bedeckt.

**39.** Implantierbare Batterieeinheit gemäß Anspruch 36, wobei die anodische Umgebung Glucoseoxidase umfasst.

**40.** Implantierbare Batterieeinheit gemäß Anspruch 39, wobei die Glucoseoxidase mit wenigstens einer Anode komplexiert ist.

**41.** Implantierbare Batterieeinheit gemäß Anspruch 36, wobei die anodische Umgebung Ammoniak umfasst.

**Revendications**

**1.** Un dispositif de pile implantable pour utilisation dans le corps d'un sujet comprenant au moins une cellule voltaïque, chacune comprenant :

- une coquille biologiquement inerte (10) définissant un compartiment interne ;
- un environnement cathodique à l'intérieur de ladite coquille (10) en contact avec ladite solution ionique ;
- un environnement anodique attaché à ladite surface externe de ladite coquille (10), en contact avec un fluide corporel dans lequel ledit environnement anodique accepte des électrons d'une réaction d'oxydation avec au moins un matériel biologique oxydable dans ledit fluide corporel ;
- un connecteur (18) connectant ledit environnement anodique et ledit environnement cathodique ;
- un composant qui fournit une résistance (20) disposé le long dudit connecteur (18) entre ledit environnement anodique et ledit environnement cathodique ; et
- au moins un pont salin (16),

**caractérisé en ce que** :

ledit compartiment interne contenant une solution ionique qui arbitre une réaction de réduction, ladite coquille (10) ayant une surface interne et une surface externe, ladite surface interne étant en contact avec ladite solution ionique ; et

ledit au moins un pont salin (16) est disposé à l'intérieur de ladite coquille (10) permettant sélectivement le passage d'ions hydrogène ou oxygène entre ledit compartiment interne et ledit liquide corporel pour compléter le circuit.

**2.** Le dispositif de pile implantable de la revendication 1 dans lequel ledit environnement cathodique comprend au moins une cathode (12).

**3.** Le dispositif de pile implantable de la revendication 1 dans lequel ledit environnement anodique comprend au moins une anode (14).

**4.** Le dispositif de pile implantable de la revendication 2 dans lequel ledit environnement anodique comprend au moins une anode (14).

**5.** Le dispositif de pile implantable de la revendication 1 dans lequel ledit matériel biologique oxydable est sélectionné dans le groupe consistant en des glucides, des cholestérols, des acides gras, des acides aminés, des polypeptides, des lipides, des hormones et des polynucléotides.

**6.** Le dispositif de pile implantable de la revendication 5 dans lequel ledit glucide est du glucose.

**7.** Le dispositif de pile implantable de la revendication 5 dans lequel ladite hormone est sélectionnée dans le groupe consistant en un oestrogène, une progestérone, une testostérone, une hormone de croissance et une hormone thyroïdienne.

**8.** Le dispositif de pile implantable de la revendication 5 dans lequel ledit polypeptide est un antigène prostate-spécifique ou un polypeptide cancer-spécifique.

**9.** Le dispositif de pile implantable de la revendication 1 dans lequel ladite solution ionique comprend un complexe diamine argent $[Ag(NHs)_2J^+$, de l'argent, du cuivre ou des sels de fer.

**10.** Le dispositif de pile implantable de la revendication 1 dans lequel ladite cellule voltaïque comprend une pluralité de cathodes (12).

**11.** Le dispositif de pile implantable de la revendication 1 dans lequel ladite cellule voltaïque comprend une pluralité d'anodes (14).

**12.** Le dispositif de pile implantable de la revendication 11 dans lequel ladite cellule voltaïque comprend une pluralité de cathodes (12).

**13.** Le dispositif de pile implantable de la revendication 1 dans lequel ladite coquille comprend une pluralité de ponts salins (16).

**14.** Le dispositif de pile implantable de la revendication 1 dans lequel ledit connecteur (18) est un fil.

**15.** Le dispositif de pile implantable de la revendication 1 dans lequel ladite cathode (12) est un métal non-magnétique.

**16.** Le dispositif de pile implantable de la revendication 2 dans lequel ladite cathode (12) est sélectionnée dans le groupe consistent en de l'argent, de l'aluminium, du plomb, du magnésium, du platine, de l'or, de l'oxyde d'étain, du dioxyde de titane, du tungstène, des alliages non-magnétiques, des semi-conducteurs, des semi-métaux et des complexes organométalliques.

**17.** Le dispositif de pile implantable de la revendication 1 dans lequel ladite coquille (10) est formée à partir d'un matériel imperméable.

**18.** Le dispositif de pile implantable de la revendication 1 dans lequel ladite coquille (10) est formée à partir un matériel semi-perméable.

**19.** Le dispositif de pile implantable de la revendication 1 comprenant en outre une barrière inerte ayant une perméabilité sélective pour exclure l'incrustation de molécules dans lequel ladite barrière recouvre ladite anode.

**20.** Le dispositif de pile implantable de la revendication 19 dans lequel ladite barrière comprend une couche qui recouvre ladite surface externe de ladite coquille.

**21.** Le dispositif de pile implantable de la revendication 1 dans lequel ledit composant qui fournit une résistance (20) est sélectionné dans le groupe consistant en une microradio ou un microprocesseur.

**22.** Le dispositif de pile implantable de la revendication 3 dans lequel ladite anode est complexée avec des molécules d'ammoniac (NH3).

**23.** Le dispositif de pile implantable de la revendication 3 dans lequel ladite anode (14) comprend une enzyme qui oxyde ledit matériel biologique oxydable.

**24.** Le dispositif de pile implantable de la revendication 23 dans lequel ladite enzyme est sélectionnée dans le groupe consistant en une glucose oxydase, une cholestérol oxydase, une hexose oxydase, une acide-1-amino oxydase, une acide-D-amino oxydase, uncase, une lactate oxydase, une choline oxydase, une alcool oxydase, uncase, une xanthine oxydase, une bilirubine oxydase, une glutamate oxydase, et une polyamine oxydase.

**25.** Le dispositif de pile implantable de la revendication 1 comprenant en outre un environnement métabolique disposé sur la surface externe de ladite coquille ou superposé audit environnement anodique dans lequel l'environnement métabolique comprend une pluralité d'enzymes et cofacteurs métaboliques qui oxydent une pluralité de matériels biologiques oxydables.

**26.** Le dispositif de pile implantable de la revendication 25 dans lequel ledit environnement métabolique comprend des enzymes et des cofacteurs métaboliques sélectionnés dans le groupe consistant en des oxydoréductases, des transférases, des hydrolases, des lyases, des isomérases, des ligases, des coenzymes, des cofacteurs et des groupes prosthétiques.

**27.** Dispositif de pile implantable de la revendication 26 dans lequel ladite pluralité d'enzymes et de cofacteurs métaboliques comprend des oxydases, des réductases, des déshydrogénases, des synthases, des enzymes du cycle de l'acide citrique, une $NAD^+$/NADH, une FMN/FMNH2, des NADH déshydrogénases, une FAD/FADH2, une fumarate/succinate, une succinate déshydrogénase, une acyl-CoA déshydrogénase, une ubiquinone.

**28.** Le dispositif de pile implantable de la revendication 27 dans lequel ledit environnement cathodique comprend des cytochromes b, c et a + $3_3$, une porphyrine contenant du fer, et une ubiquinone.

**29.** Le dispositif de pile implantable de la revendication 25 dans lequel une seule couche comprend ledit environnement métabolique et ledit environnement anodique.

**30.** Le dispositif de pile implantable de la revendication 25 dans lequel ledit environnement métabolique est disposé en une couche sur une portion externe dudit environnement anodique et est en contact avec ledit fluide corporel, dans lequel ledit environnement anodique est séparé dudit environnement métabolique par une couche d'arrêt imperméable, dans lequel ladite couche d'arrêt comprend des canaux ioniques qui sont connectés opérationnellement à des récepteurs pour une substance à analyser, lesdits récepteurs s'étendant depuis lesdits canaux vers ledit fluide corporel, dans lequel la fixation de ladite substance à analyser audit récepteur cause l'ouverture desdits canaux et rend possible un flux d'électron vers ledit environnement anodique.

**31.** Le dispositif de pile implantable de la revendication 28 dans lequel ledit environnement anodique comprend une couche d'arrêt imperméable entre ledit environnement anodique et ledit fluide corporel, dans lequel ladite couche d'arrêt comprend des canaux ioniques qui sont connectés opérationnellement à des récepteurs pour une substance à analyser, lesdits récepteurs s'étendant depuis lesdits canaux vers le fluide corporel, dans lequel la fixation de ladite substance à analyser audit récepteur cause l'ouverture desdits canaux et rend possible un flux dudit matériel biologique oxydable dans ledit environnement anodique.

**32.** Le dispositif de pile implantable selon la revendication 1, dans lequel ledit environnement anodique comprend des donneurs d'électrons, des antioxydants ou des réducteurs actifs ; un environnement cathodique.

**33.** Le dispositif de pile implantable selon la revendication 1 dans lequel lesdits donneurs d'électrons comprennent une NADH, une $FADH_2$, et une $FMNH_2$-.

**34.** Le dispositif de pile implantable selon la revendication 1, dans lequel lesdits antioxydants comprennent le glutathion ou l'ascorbate.

**35.** Le dispositif de pile implantable selon la revendication 1, dans lequel lesdits réducteurs actifs comprennent le borohydrure de sodium.

**36.** Le dispositif de pile implantable de la revendication 1 dans lequel le matériel oxydable est du glucose, comprenant en outre un dispositif de détection, dans lequel ledit dispositif de détection détecte un signal depuis ledit premier composant et fournit une valeur qui corrèle avec le niveau de glucose sanguin dans ledit sujet.

**37.** Le dispositif de pile implantable de la revendication 36 dans lequel ledit dispositif de détection est un ampèremètre.

**38.** Le dispositif de pile implantable de la revendication 36 comprenant en outre une couche barrière inerte ayant une perméabilité sélective aux petits ions qui couvrent ladite anode.

**39.** Le dispositif de pile implantable de la revendication 36 dans lequel ledit environnement anodique comprend une glucose oxydase.

**40.** Le dispositif de pile implantable de la revendication 39 dans lequel ladite glucose oxydase est complexée avec au moins une anode.

**41.** Le dispositif de pile implantable de la revendication 36 dans lequel ledit environnement anodique comprend de l'ammoniac.

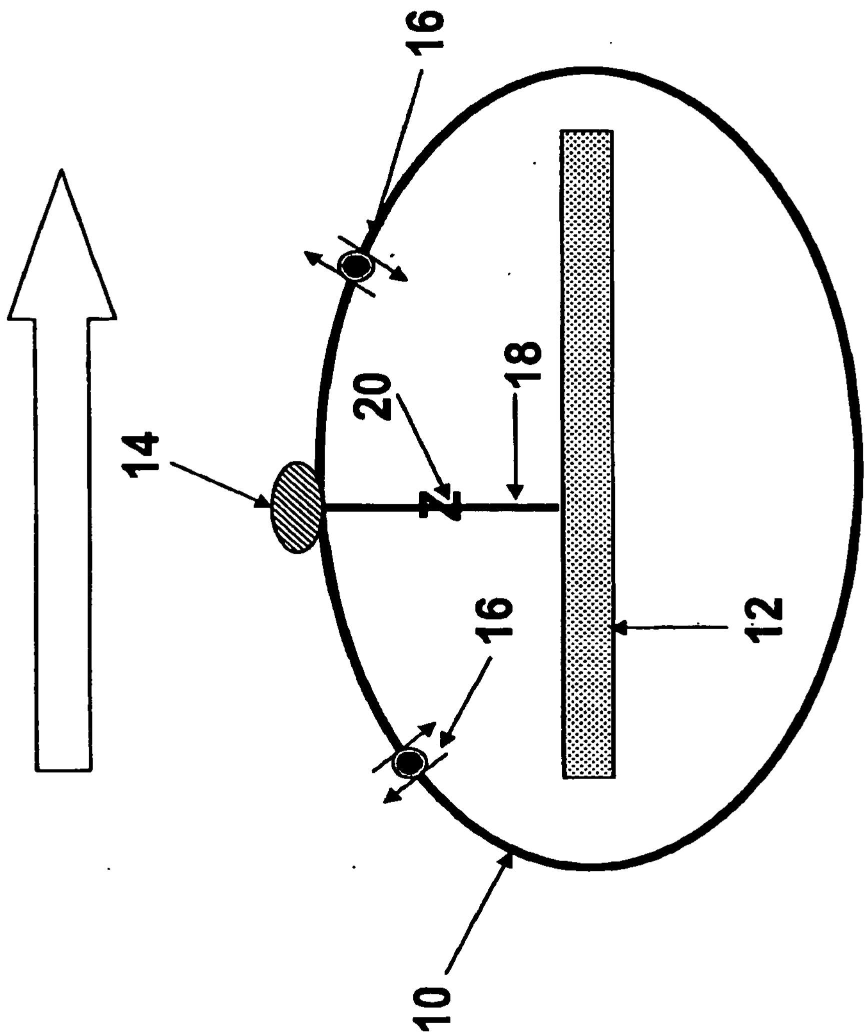


Figure 1

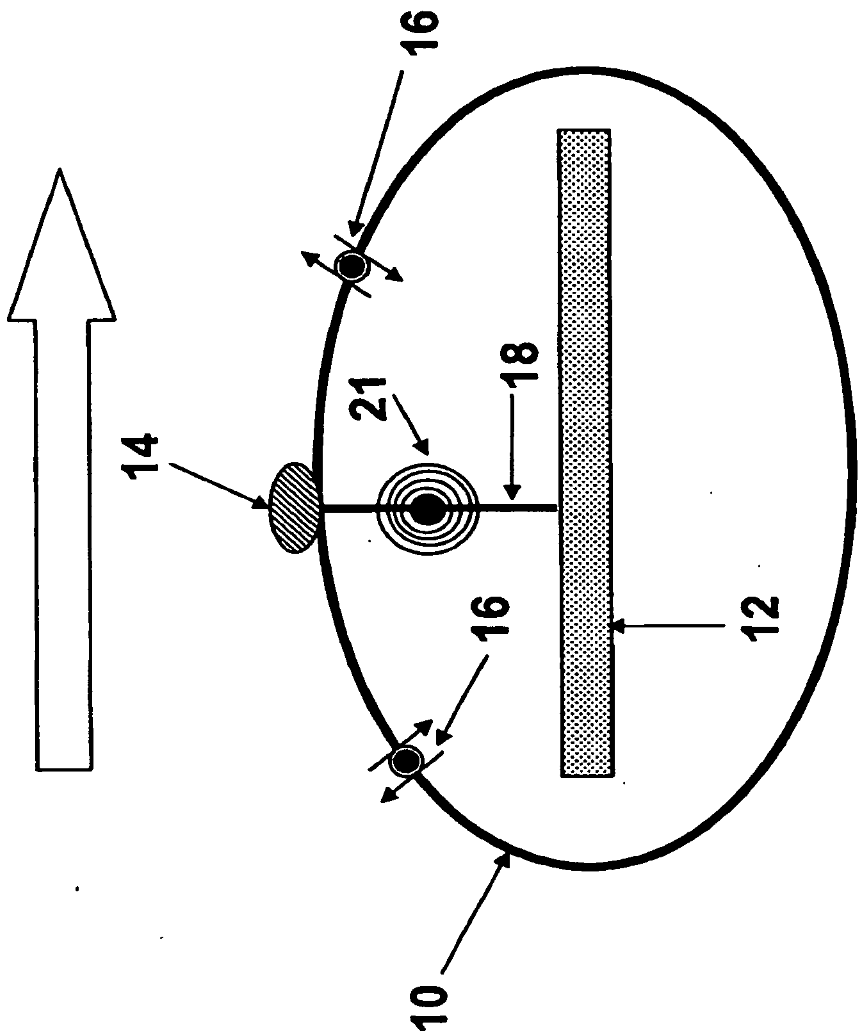


Figure 2

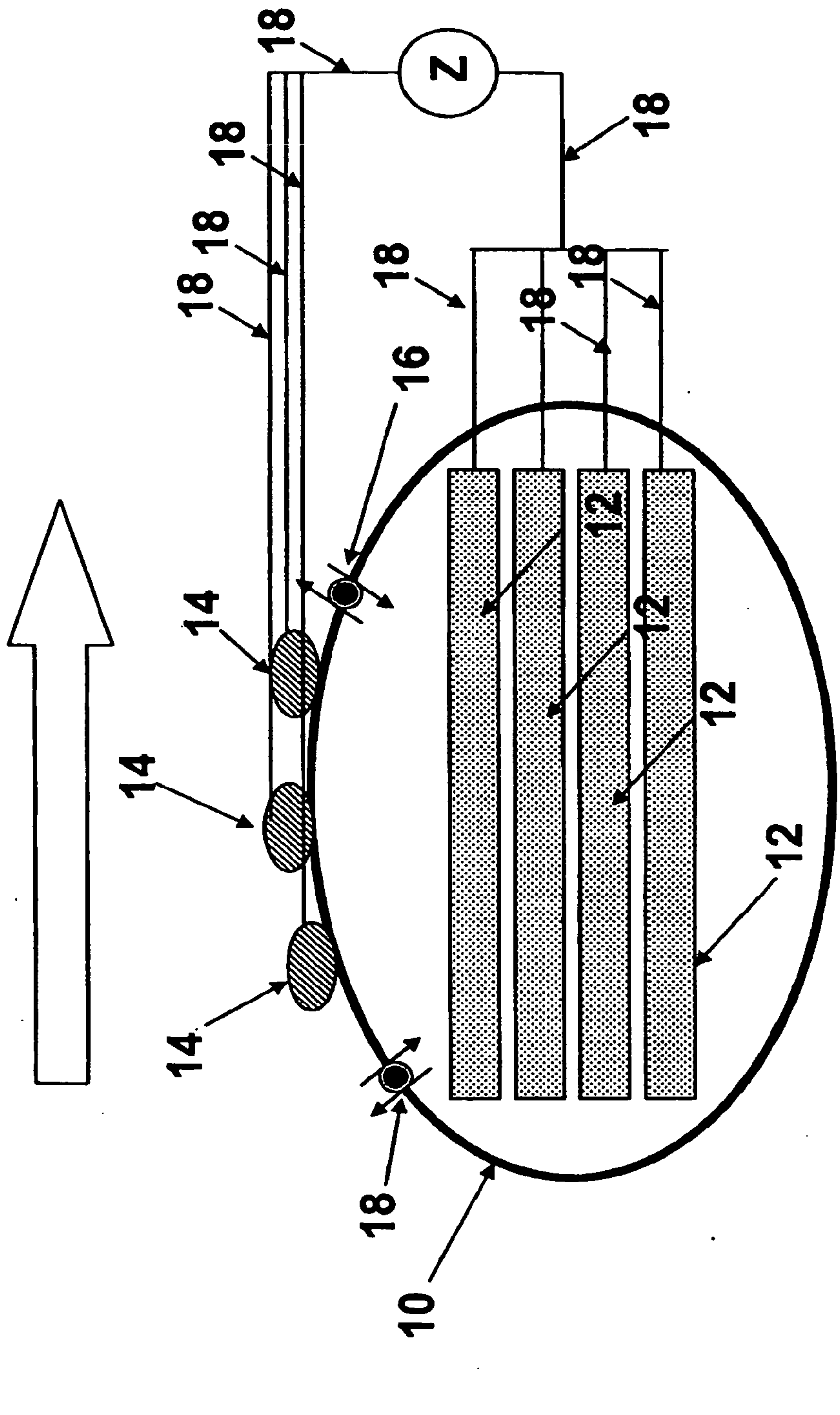


Figure 3

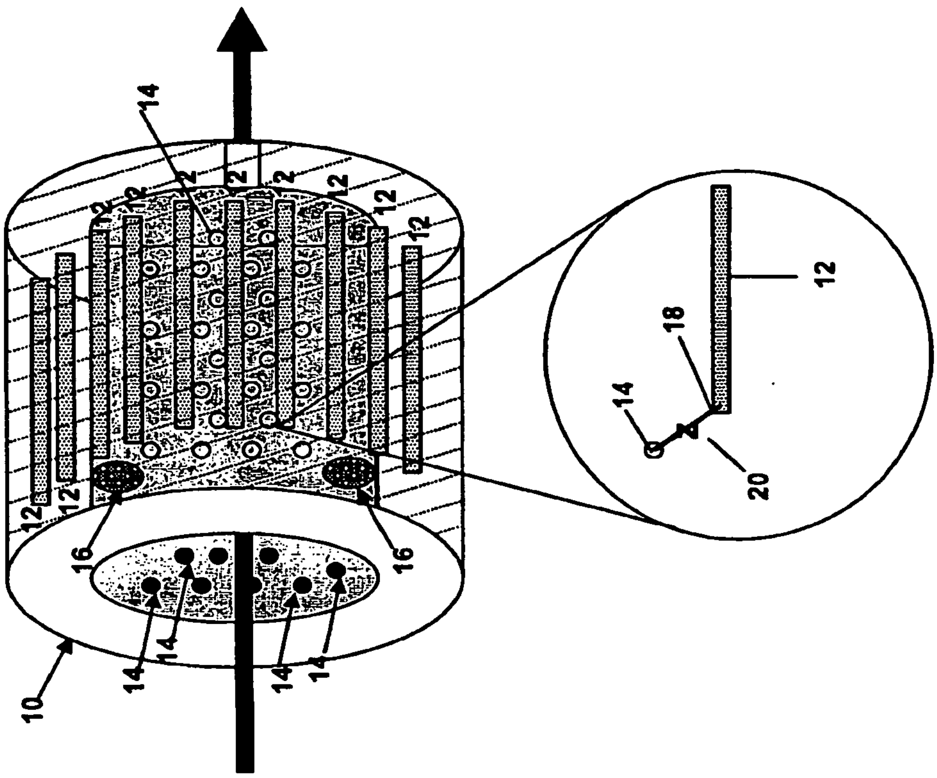


Figure 4

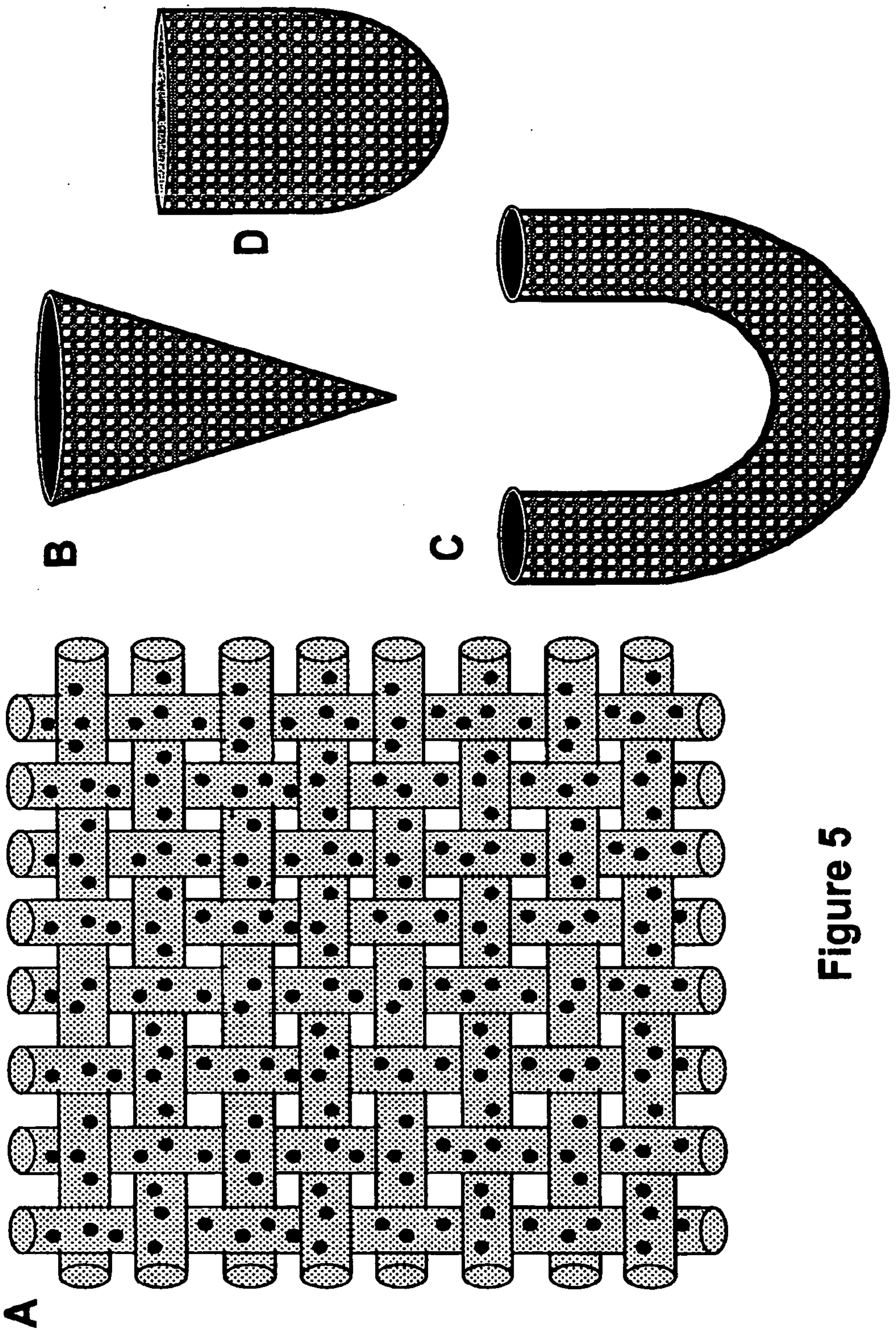


Figure 5

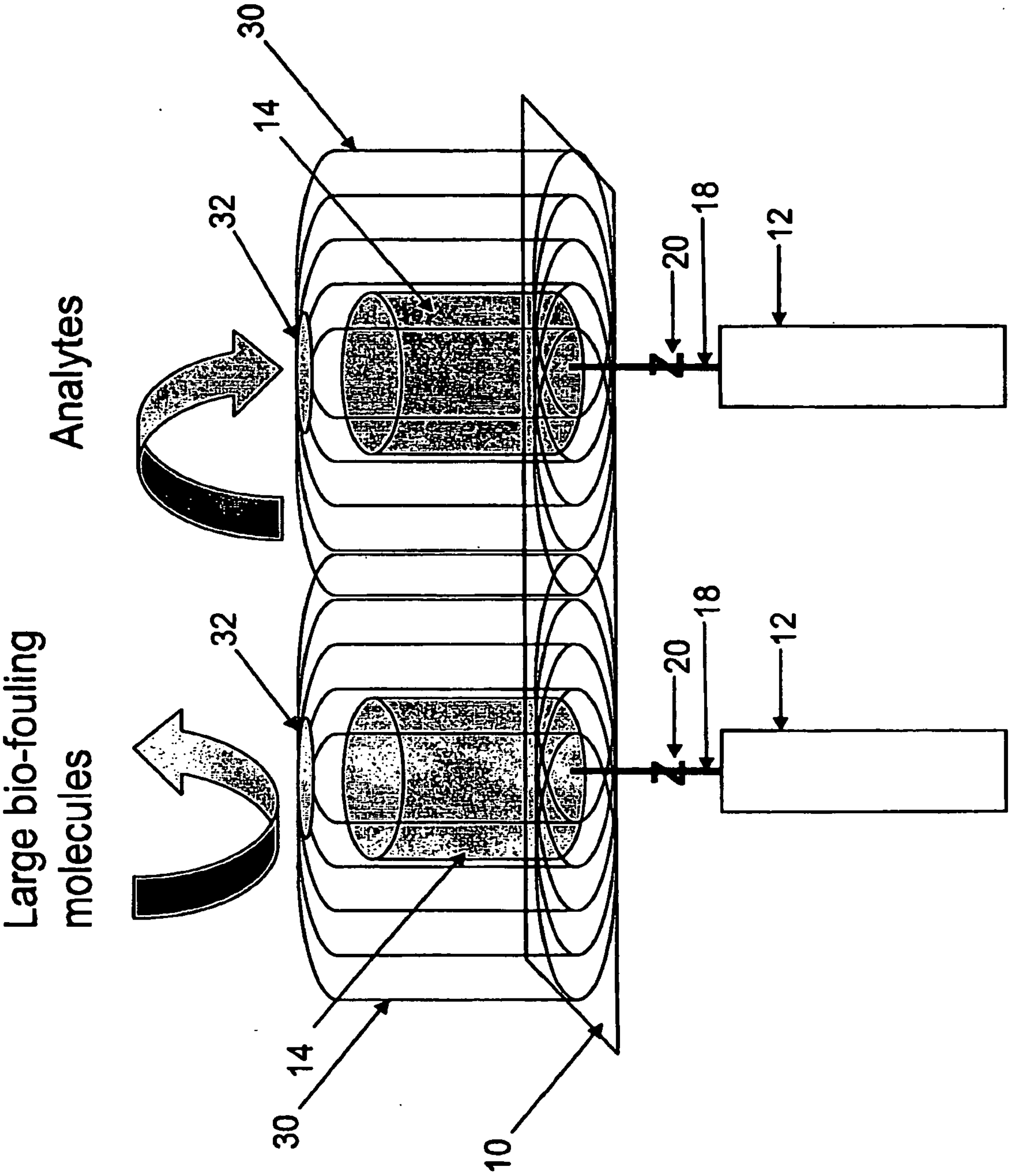
Large bio-fouling molecules
Analytes
32
32
14
14
30
30
10
20
18
12
20
18
12

Figure 6

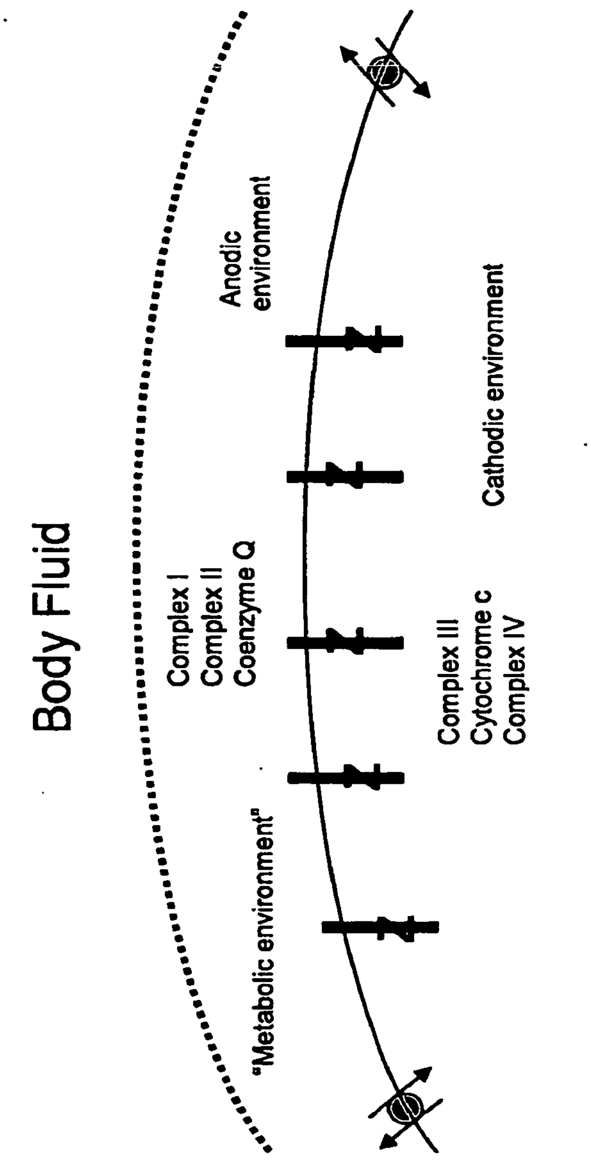
Body Fluid
"Metabolic environment"
Complex I
Complex II
Coenzyme Q
Anodic
environment
Complex III
Cytochrome c
Complex IV
Cathodic environment

Figure 7

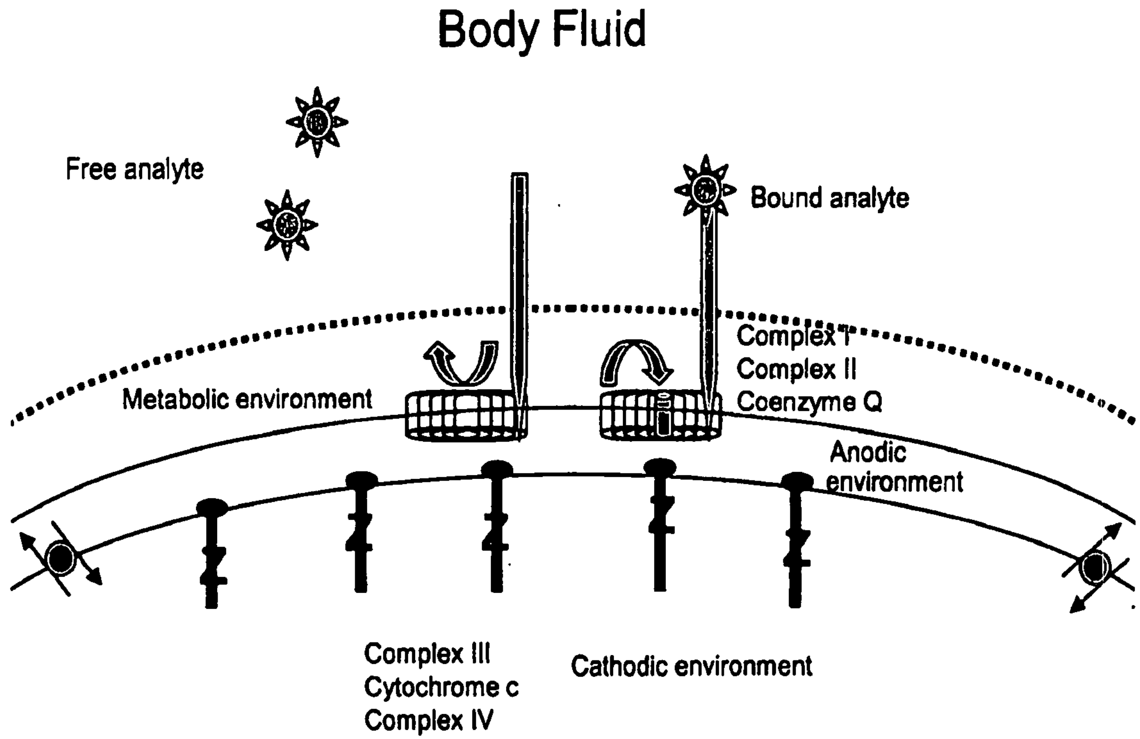
Body Fluid
Free analyte
Bound analyte
Metabolic environment
Complex I
Complex II
Coenzyme Q
Anodic environment
Complex III
Cytochrome c
Complex IV
Cathodic environment

Figure 8

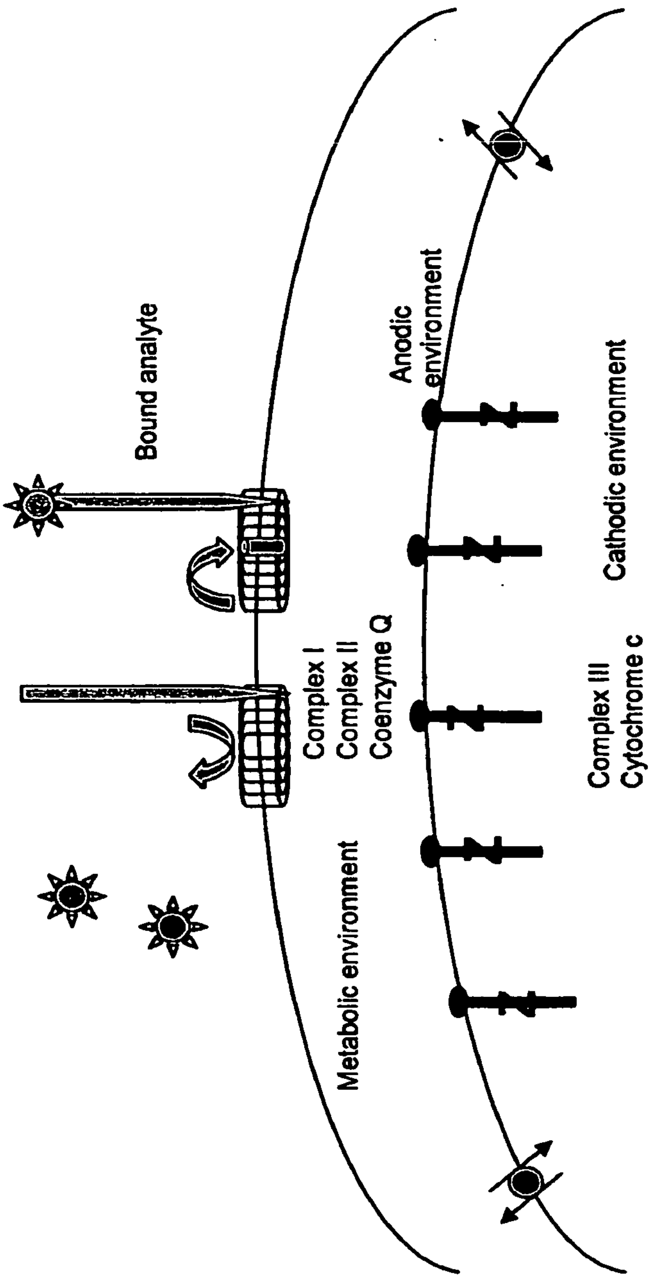
Bound analyte
Anodic environment
Cathodic environment
Complex I
Complex II
Coenzyme Q
Complex III
Cytochrome c
Complex IV
Metabolic environment

**Figure 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 79768006 P
- EP 1541191 A
- US 200502457299 A
- US 6585644 B


### Non-patent literature cited in the description


- **CHAMPE, P. ; HARVEY, R.** LIPPINCOTT'S ILLUSTRATED REVIEWS. Lippincott, Williams and Wilkins, 1994
- **BROWN et al.** CHEMISTRY: THE CENTRAL SCIENCE. Pearson Education, 2006